# EUROPEAN PATENT SPECIFICATION

(11) **EP 4 228 552 B1**
(45) Date of publication and mention of the grant of the patent: **25.03.2026**
(21) Application number: 21806887.2
(22) Date of filing: 13.10.2021
(51) Int. Cl.: A61F 2/24, A61M 25/00

(54) **EXPANDABLE SHEATH WITH RADIOPAQUE FEATURES**
EXPANDIERBARE HÜLLE MIT RÖNTGENDICHTEN MERKMALEN
GAINE EXTENSIBLE À CARACTÉRISTIQUES RADIO-OPAQUES

(30) Priority: 14.10.2020 US 202063091722 P
(43) Date of publication of application: 23.08.2023
(73) Proprietor: Edwards Lifesciences Corporation, Irvine, CA 92614-5688 (US)
(72) Inventor: NEUMANN, Yair A., 3079892 Caesarea (IL); DAVIDESKO, Amir, 3780800 Binyamina Givat Ada (IL); HICKS, Kristen, Irvine, CA 92614 (US); SHITRIT, Roy, 3079892 Caesarea (IL)
(74) Representative: Eisenführ Speiser
(86) International application number: PCT/US2021/054788
(87) International publication number: WO 2022/081710

(56) References cited:
- WO-A2-2021/071960
- US-A1- 2012 083 877

## Description

### RELATED APPLICATIONS

This application claims the benefit of U.S. Provisional Application number 63/091,722, filed October 14, 2020.

### FIELD

The present application relates to expandable introducer sheaths for prosthetic devices such as transcatheter heart valves, and methods of making the same.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (*e.g.*, the femoral artery). An introducer sheath generally has an elongated sleeve that is inserted into the vasculature and a housing that contains one or more sealing valves that allow a delivery apparatus to be placed in fluid communication with the vasculature with minimal blood loss. Such introducer sheaths may be radially expandable. However, such sheaths tend to have complex mechanisms, such as ratcheting mechanisms that maintain the sheath in an expanded configuration once a device with a larger diameter than the sheath's original diameter is introduced. Existing expandable sheaths can also be prone to axial elongation as a consequence of the application of longitudinal force attendant to passing a prosthetic device through the sheath. Such elongation can cause a corresponding reduction in the diameter of the sheath, increasing the force required to insert the prosthetic device through the narrowed sheath.

US 2012/0083877 A1 discloses an expandable sheath for use in conjunction with a catheter assembly to introduce a prosthetic device, such as a heart valve, into a patient. Such a sheath allows for temporary expansion of a portion of the introducer sheath to accommodate the delivery apparatus, followed by a return to the original diameter once the prosthetic device passes through. The sheath comprises inner and outer layers, where a folded portion of the inner layer extends through a slit in the outer layer and a portion of the outer layer overlaps the folded portion of the inner layer. The sheath includes an elastic outer cover positioned outside the outer layer.

Accordingly, there remains a need in the art for an improved introducer sheath for endovascular systems used for implanting valves and other prosthetic devices.

### SUMMARY

Expandable sheaths with one or more radiopaque features are disclosed herein. The radiopaque features improve visualization of the sheath within the body during a medical procedure.

The claimed invention is defined in independent claim 1 and relates to an expandable sheath for deploying a medical device. Preferred configurations of the claimed invention are defined in dependent claims 2 to 15.

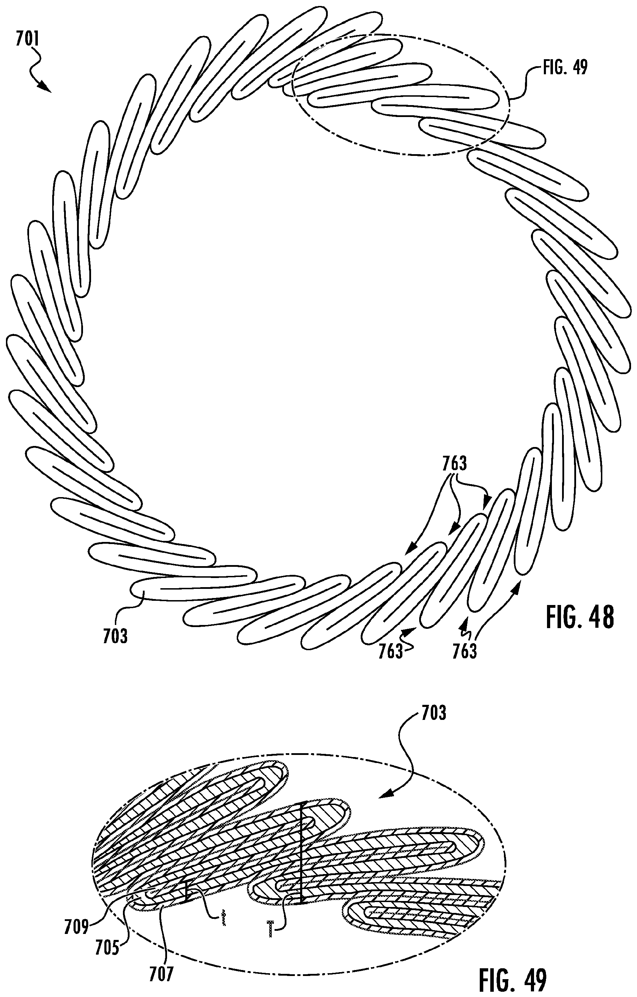

Certain aspects and/or particularly preferred configurations of the claimed invention are discussed hereafter for example in conjunction with Figs. 48-54. Also described herein are related aspects, examples, embodiments and arrangements useful for understanding the claimed invention.

In some aspects, the sheath wall includes a tubular foil layer that functions as the radiopaque feature. The sheath wall also includes a tubular first polymeric layer that defines an inner lumen. The tubular, radiopaque foil layer is positioned radially outward of the first polymeric layer and extends circumferentially around the first polymeric layer. In a collapsed state, the sheath wall has a plurality of longitudinally extending folds that create a plurality of circumferentially spaced ridges and a plurality of circumferentially spaced valleys. When a medical device passes through the inner lumen of the sheath, it applies an outward radial force on the sheath wall. This outward force causes the sheath wall to expand radially from the collapsed state to an expanded state by at least partially unfolding the plurality of longitudinally extending folds such that the ridges and valleys level out. Unfolding the plurality of longitudinally extending folds causes a decrease in the total thickness of the sheath wall. In some aspects, the decrease in the sheath wall thickness can be detected by an imaging modality as a decrease in radiopacity. In some aspects, the sheath wall includes a second polymeric layer positioned radially outward of the foil layer. The foil layer can be encapsulated or sandwiched between the first and second polymeric layers.

Some expandable sheaths disclosedd herein can include a first polymeric layer, a braided layer positioned radially outward of the first polymeric layer (the braided layer comprising a plurality of filaments braided together), a radiopaque feature positioned radially outward of the first polymeric layer, and a second polymeric layer positioned radially outward of the braided layer and the radiopaque feature. The second polymeric layer is coupled to the first polymeric layer such that the braided layer and the radiopaque feature are encapsulated between the first and second polymeric layers. When a medical device is passed through the sheath, the diameter of the sheath expands from a first diameter to a second diameter around the medical device. Some embodiments can include one or more additional radiopaque features. The one or more additional radiopaque features may be of the same type as the first radiopaque feature, or of one or more different types of radiopaque features.

### BRIEF DESCRIPTION OF THE DRAWINGS

FIG. 1 illustrates a delivery system for a cardiovascular prosthetic device, according to one aspect.
FIG. 2 illustrates an expandable sheath that can be used in combination with the delivery system of FIG. 1, according to one aspect.
FIG. 3 is a magnified view of a portion of the expandable sheath of FIG. 2.
FIG. 4 is a side elevation cross-sectional view of a portion of the expandable sheath of FIG. 2.
FIG. 5A is a magnified view of a portion of the expandable sheath of FIG. 2 with the outer layer removed for purposes of illustration.
FIG. 5B is a magnified view of a portion of the braided layer of the sheath of FIG. 2.
FIG. 6 is a magnified view of a portion of the expandable sheath of FIG. 2 illustrating expansion of the sheath as a prosthetic device is advanced through the sheath.
FIG. 7 is a magnified, partial cross-sectional view illustrating the constituent layers of the sheath of FIG. 2 disposed on a mandrel.
FIG. 8 is a magnified view illustrating another aspect of an expandable sheath.
FIG. 9 is a cross-sectional view of an apparatus that can be used to form an expandable sheath, according to one aspect.
FIGS. 10A-10D illustrate another aspect of a braided layer in which the filaments of the braided layer are configured to buckle when the sheath is in a radially collapsed state.
FIG. 11 shows a side cross-sectional view of an assembly of an expandable sheath with a vessel dilator.
FIG. 12 shows the vessel dilator of the assembly aspect of FIG. 11.
FIG. 13 shows a side view of another assembly aspect including an expandable sheath and a vessel dilator.
FIG. 14 shows a side view of the assembly aspect of FIG. 13, with the vessel dilator pushed partially away from the expandable sheath.
FIG. 15 shows a side view of the assembly aspect of FIG. 13, with the vessel dilator pushed fully away from the expandable sheath.
FIG. 16 shows a side view of the assembly aspect of FIG. 13, with the vessel dilator being retracted into the expandable sheath.
FIG. 17 shows a side view of the assembly aspect of FIG. 13, with the vessel dilator being retracted further into the expandable sheath.
FIG. 18 shows a side view of the assembly aspect of FIG. 13, with the vessel dilator being fully retracted into the expandable sheath.
FIG. 19 shows a side cross sectional view of another assembly aspect including an expandable sheath and a vessel dilator.
FIG. 20 illustrates and aspect of a vessel dilator that may be used in combination with the expandable sheaths described herein.
FIG. 21 illustrates an aspect of a vessel dilator that may be used in combination with the expandable sheaths described herein.
FIG. 22 shows a side view with a cutaway to cross section of an aspect of an expandable sheath having an outer cover and an overhang.
FIG. 23 shows an example aspect of a outer cover having longitudinal scorelines.
FIG. 24 illustrates an end portion of an aspect of a braided layer of an expandable sheath.
FIG. 25A illustrates a perspective view of a roller-based crimping mechanism aspect for crimping an expandable sheath.
FIG. 25B illustrates a side view of a disc-shaped roller and connector of the crimping mechanism shown in FIG. 25A.
FIG. 25C illustrates a top view of a disc-shaped roller and connector of the crimping mechanism shown in FIG. 25A.
FIG. 26 shows an aspect of a device for crimping an elongated expandable sheath. The encircled portion of the device is magnified in the inset at the left side of the picture.
FIG. 27 shows an aspect of an expandable sheath having an inner layer with scorelines.
FIG. 28 shows an additional aspect of a braided layer of an expandable sheath.
FIG. 29 shows a perspective view of an additional expandable sheath aspect.
FIG. 30 shows a perspective view of the aspect of FIG. 29 with the outer heat shrink tubing layer partially torn away from the inner sheath layers.
FIG. 31 shows a side view of a sheath aspect prior to movement of a delivery system therethrough.
FIG. 32 shows a side view of a sheath aspect as a delivery system moves through, splitting the heat shrink tubing layer.
FIG. 33 shows a side view of a sheath aspect with the delivery system fully moved through, the heat shrink tubing layer fully split along the length of the sheath.
FIG. 34 shows a perspective view of a sheath aspect having a distal end portion folded around an introducer.
FIG. 35 shows an enlarged, cross sectional view of the distal end portion folded around the introducer.
FIG. 36 shows a cross section of an additional expandable sheath aspect.
FIG. 37 shows an aspect of a cushioning layer.
FIG. 38 shows another aspect of a cushioning layer.
FIG. 39 shows a side view of an additional expandable sheath aspect.
FIG. 40 shows a longitudinal cross section of the aspect of FIG. 39.
FIG. 41 shows a transverse cross section of an additional expandable sheath aspect.
FIG. 42 shows a partial longitudinal cross section of an additional expandable sheath aspect.
FIG. 43 shows a transverse cross section of an additional expandable sheath aspect in an expanded state.
FIG. 44 shows a transverse cross section of the expandable sheath aspect of FIG. 43 during the crimping process.
FIG. 45 shows a perspective view of a sheath aspect similar to the sheath of FIG. 43, in the expanded state.
FIG. 46 shows a perspective view of a sheath aspect similar to the sheath of FIG. 43, in the folded and compressed state.
FIG. 47 shows an additional aspect of a braided layer.
FIG. 48 shows a simplified cross section of a sheath wall adjacent the distal tip of the sheath, the sheath wall having a plurality of longitudinally extending folds.
FIG. 49 shows a detailed cross section of the sheath wall of FIG. 48, showing polymeric layers and a radiopaque foil layer.
FIG. 50 shows a simplified cross section of a sheath wall adjacent the distal tip of the sheath, the sheath wall having a plurality of longitudinally extending folds.
FIG. 51 shows a cross section of a sheath wall including a radiopaque foil layer.
FIG. 52 shows another cross section of a sheath wall including a radiopaque foil layer.
FIG. 53 shows another cross section of a sheath wall including a radiopaque foil layer.
FIG. 54 shows another cross section of a sheath wall including a radiopaque foil layer.
FIG. 55 shows an aspect of an expandable sheath including a radiopaque cord.
FIG. 56 shows an aspect of an expandable sheath including a radiopaque feature positioned distal to the distal end of a braided layer.
FIG. 57 shows an aspect of an expandable sheath including radiopaque markers within cells of a braided layer.
FIG. 58 shows an aspect of an expandable sheath including radiopaque coils coupled to filaments of a braided layer.
FIG. 59 shows an aspect of an expandable sheath including radiopaque beads joining circumferentially adjacent distal ends of filaments of a braided layer.
FIG. 60 shows an aspect of an expandable sheath including a radiopaque cord.
FIG. 61 shows a distal portion of a braided layer with a radiopaque tube.
FIG. 62 shows an aspect wherein an external covering layer covers a distal portion of a braided layer with a radiopaque tube.
FIG. 63 shows an *in vivo* image of an expandable sheath with a radiopaque tube.

### DETAILED DESCRIPTION

The expandable introducer sheaths described herein can be used to deliver a prosthetic device through a patient's vasculature to a procedure site within the body. The sheath can be constructed to be highly expandable and collapsible in the radial direction while limiting axial elongation of the sheath and, thereby, undesirable narrowing of the lumen. In one aspect, the expandable sheath includes a braided layer, one or more relatively thin, non-elastic polymeric layers, and an elastic layer. The sheath can resiliently expand from its natural diameter to an expanded diameter as a prosthetic device is advanced through the sheath, and can return to its natural diameter upon passage of the prosthetic device under the influence of the elastic layer. In certain aspects, the one or more polymeric layers can engage the braided layer, and can be configured to allow radial expansion of the braided layer while preventing axial elongation of the braided layer, which would otherwise result in elongation and narrowing of the sheath.

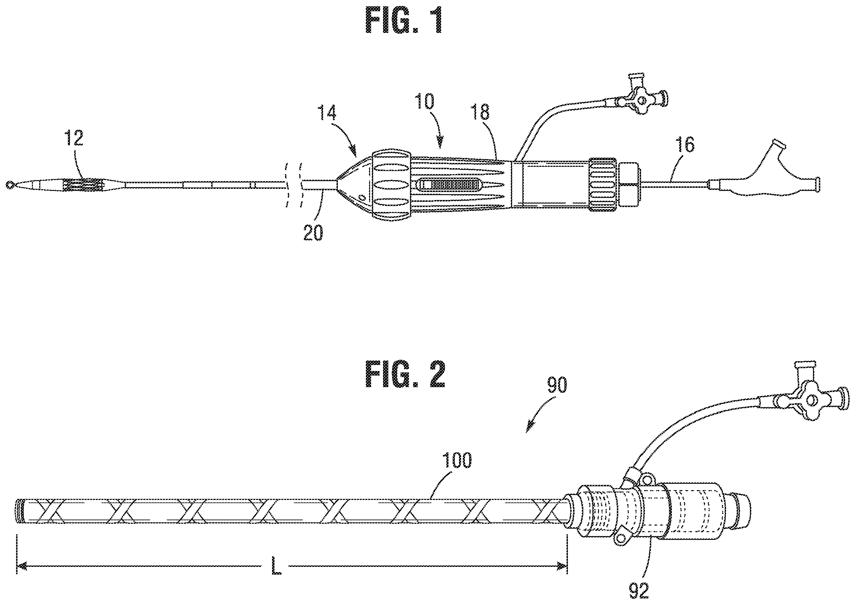

FIG. 1 illustrates a representative delivery apparatus 10 for delivering a medical device, such as a prosthetic heart valve or other prosthetic implant, to a patient. The delivery apparatus 10 is exemplary only, and can be used in combination with any of the expandable sheath aspects described herein. Likewise, the sheaths disclosed herein can be used in combination with any of various known delivery apparatuses. The delivery apparatus 10 illustrated can generally include a steerable guide catheter 14 and a balloon catheter 16 extending through the guide catheter 14. A prosthetic device, such as a prosthetic heart valve 12, can be positioned on the distal end of the balloon catheter 16. The guide catheter 14 and the balloon catheter 16 can be adapted to slide longitudinally relative to each other to facilitate delivery and positioning of a prosthetic heart valve 12 at an implantation site in a patient's body. The guide catheter 14 includes a handle 18 and an elongated guide tube or shaft 20 extending from the handle 18.

The prosthetic heart valve 12 can be delivered into a patient's body in a radially compressed configuration and radially expanded to a radially expanded configuration at the desired deployment site. In the illustrated aspect, the prosthetic heart valve 12 is a plastically expandable prosthetic valve that is delivered into the patient's body in a radially compressed configuration on a balloon of the balloon catheter 16 (as shown in FIG. 1) and then radially expanded to a radially expanded configuration at the deployment site by inflating the balloon (or by actuating another type of expansion device of the delivery apparatus). Further details regarding a plastically expandable heart valve that can be implanted using the devices disclosed herein are disclosed in U.S. Publication No. 2012/0123529. In other aspects, the prosthetic heart valve 12 can be a self-expandable heart valve that is restrained in a radially compressed configuration by a sheath or other component of the delivery apparatus and self-expands to a radially expanded configuration when released by the sheath or other component of the delivery apparatus. Further details regarding a self-expandable heart valve that can be implanted using the devices disclosed herein are disclosed in U.S. Publication No. 2012/0239142. In still other aspects, the prosthetic heart valve 12 can be a mechanically expandable heart valve that comprises a plurality of struts connected by hinges or pivot joints and is expandable from a radially compressed configuration to a radially expanded configuration by actuating an expansion mechanism that applies an expansion force to the prosthetic valve. Further details regarding a mechanically expandable heart valve that can be implanted using the devices disclosed herein are disclosed in U.S. Publication No. 2018/0153689. In still other aspects, a prosthetic valve can incorporate two or more of the above-described technologies. For example, a self-expandable heart valve can be used in combination with an expansion device to assist expansion of the prosthetic heart valve.

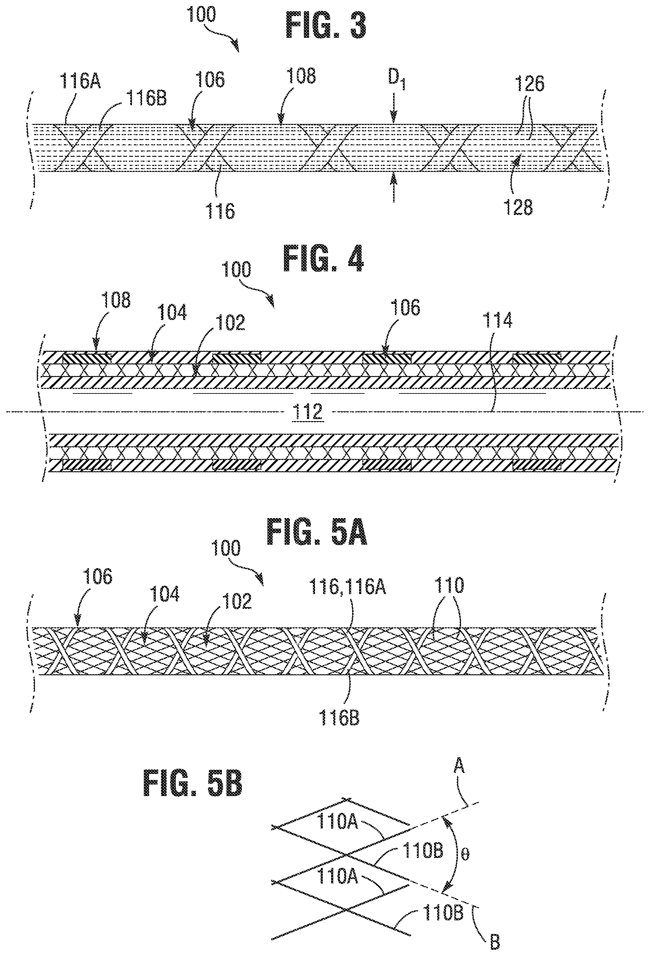

FIG. 2 illustrates an assembly 90 (which can be referred to as an introducer device or assembly) that can be used to introduce the delivery apparatus 10 and the prosthetic device 12 into a patient's body, according to one aspect. The introducer device 90 can comprise a housing 92 at a proximal end of the device and an expandable sheath 100 extending distally from the housing 92. The housing 92 can function as a handle for the device. The expandable sheath 100 has a central lumen 112 (FIG. 4) to guide passage of the delivery apparatus for the prosthetic heart valve. Generally, during use a distal end of the sheath 100 is passed through the skin of the patient and is inserted into a vessel, such as the femoral artery. The delivery apparatus 10 with its prosthetic device 12 can then be inserted through the housing 92 and the sheath 100, and advanced through the patient's vasculature to the treatment site, where the implant is to be delivered and implanted within the patient. In certain aspects, the housing 92 can include a hemostasis valve that forms a seal around the outer surface of the guide catheter 14 once inserted through the housing to prevent leakage of pressurized blood.

In alternative aspects, the introducer device 90 need not include a housing 92. For example, the sheath 100 can be an integral part of a component of the delivery apparatus 10, such as the guide catheter. For example, the sheath can extend from the handle 18 of the guide catheter. Additional examples of introducer devices and expandable sheaths can be found in U.S. Patent Application No. 16/378,417 and U.S. Provisional Patent Application No. 62/912,569.

FIG. 3 illustrates the expandable sheath 100 in greater detail. With reference to FIG. 3, the sheath 100 can have a natural, unexpanded outer diameter D₁. In certain aspects, the expandable sheath 100 can comprise a plurality of coaxial layers extending along at least a portion of the length L of the sheath (FIG. 2). For example, with reference to FIG. 4, the expandable sheath 100 can include a first layer 102 (also referred to as an inner layer), a second layer 104 disposed around and radially outward of the first layer 102, a third layer 106 disposed around and radially outward of the second layer 104, and a fourth layer 108 (also referred to as an outer layer) disposed around and radially outward of the third layer 106. In the illustrated configuration, the inner polymeric layer 102 can define the central lumen 112 of the sheath extending along a central axis 114.

Referring to FIG. 3, when the sheath 100 is in an unexpanded state, the inner polymeric layer 102 and/or the outer layer 108 can form longitudinally extending folds or creases such that the surface of the sheath comprises a plurality of ridges 126 (also referred to herein as "longitudinally extending folds"). The ridges 126 can be circumferentially spaced apart from each other by longitudinally extending valleys 128. When the sheath expands beyond its natural diameter D₁, the ridges 126 and the valleys 128 can level out or be taken up as the surface radially expands and the circumference increases, as further described below. When the sheath collapses back to its natural diameter, the ridges 126 and valleys 128 can reform.

In certain aspects, the inner polymeric layer 102 and/or the outer layer 108 can comprise a relatively thin layer of polymeric material. For example, in some aspects the thickness of the inner polymeric layer 102 can be from 0.01 mm to 0.5 mm, 0.02 mm to 0.4 mm, or 0.03 mm to 0.25 mm. In certain aspects, the thickness of the outer layer 108 can be from 0.01 mm to 0.5 mm, 0.02 mm to 0.4 mm, or 0.03 mm to 0.25 mm.

In certain examples, the inner polymeric layer 102 and/or the outer layer 108 can comprise a lubricious, low-friction, and/or relatively non-elastic material. In particular aspects, the inner polymeric layer 102 and/or the outer layer 108 can comprise a polymeric material having a modulus of elasticity of 400 MPa or greater. Exemplary materials can include ultra-high-molecular-weight polyethylene (UHMWPE) (e.g., Dyneema^{®}), high-molecular-weight polyethylene (HMWPE), or polyether ether ketone (PEEK). With regard to the inner polymeric layer 102 in particular, such low coefficient of friction materials can facilitate passage of the prosthetic device through the central lumen 112. Other suitable materials for the inner and outer layers can include polytetrafluoroethylene (PTFE), expanded polytetrafluoroethylene (ePTFE), ethylene tetrafluoroethylene (ETFE), nylon, polyethylene, polyether block amide (*e.g.*, Pebax), and/or combinations of any of the above. Some aspects of a sheath 100 can include a lubricious liner on the inner surface of the inner polymeric layer 102. Examples of suitable lubricious liners include materials that can further reduce the coefficient of friction of the inner polymeric layer 102, such as PTFE, polyethylene, polyvinylidine fluoride, and combinations thereof. Suitable materials for a lubricious liner also include other materials desirably having a coefficient of friction of 0.1 or less.

Additionally, some aspects of the sheath 100 can include an exterior hydrophilic coating on the outer surface of the outer layer 108. Such a hydrophilic coating can facilitate insertion of the sheath 100 into a patient's vessel, reducing potential damage. Examples of suitable hydrophilic coatings include the Harmony^{™} Advanced Lubricity Coatings and other Advanced Hydrophilic Coatings available from SurModics, Inc., Eden Prairie, MN. DSM medical coatings (available from Koninklijke DSM N.V, Heerlen, the Netherlands), as well as other hydrophilic coatings (*e.g.*, PTFE, polyethylene, polyvinylidine fluoride), are also suitable for use with the sheath 100. Such hydrophilic coatings may also be included on the inner surface of the inner polymeric layer 102 to reduce friction between the sheath and the delivery system, thereby facilitating use and improving safety. In some aspects, a hydrophobic coating, such as Perylene, may be used on the outer surface of the outer layer 108 or the inner surface of the inner polymeric layer 102 in order to reduce friction.

In certain aspects, the second layer 104 can be a braided layer. FIGS. 5A and 5B illustrate the sheath 100 with the outer layer 108 removed to expose the elastic layer 106. With reference to FIGS. 5A and 5B, the braided layer 104 can comprise a plurality of members or filaments 110 (e.g., metallic or synthetic wires or fibers) braided together. The braided layer 104 can have any desired number of filaments 110, which can be oriented and braided together along any suitable number of axes. For example, with reference to FIG. 5B, the filaments 110 can include a first set of filaments 110A oriented parallel to a first axis A, and a second set of filaments 110B oriented parallel to a second axis B. The filaments 110A and 110B can be braided together in a biaxial braid such that filaments 110A oriented along axis A form an angle θ with the filaments 110B oriented along axis B. In certain aspects, the angle θ can be from 5° to 70°, 10° to 60°, 10° to 50°, or 10° to 45°. In the illustrated aspect, the angle θ is 45°. In other aspects, the filaments 110 can also be oriented along three axes and braided in a triaxial braid, or oriented along any number of axes and braided in any suitable braid pattern.

The braided layer 104 can extend along substantially the entire length L of the sheath 100, or alternatively, can extend only along a portion of the length of the sheath. In particular aspects, the filaments 110 can be wires made from a superelastic metal (e.g., Nitinol, stainless steel, etc.), or any of various polymers or polymer composite materials, such as carbon fiber. In certain aspects, the filaments 110 can be round, and can have a diameter of from 0.01 mm to 0.5 mm, 0.03 mm to 0.4 mm, or 0.05 mm to 0.25 mm. In other aspects, the filaments 110 can have a flat cross-section with dimensions of 0.01 mm x 0.01 mm to 0.5 mm x 0.5 mm, or 0.05 mm x 0.05 mm to 0.25 mm x 0.25 mm. In one aspect, filaments 110 having a flat cross-section can have dimensions of 0.1 mm x 0.2 mm. However, other geometries and sizes are also suitable for certain aspects. If braided wire is used, the braid density can be varied. Some aspects have a braid density of from ten picks per inch to eighty picks per inch, and can include eight wires, sixteen wires, or up to fifty-two wires in various braid patterns. In other aspects, layer 104 can be laser cut from a tube, or laser-cut, stamped, punched, etc., from sheet stock and rolled into a tubular configuration. The layer 104 can also be woven or knitted, as desired.

The third layer 106 can be a resilient, elastic layer (also referred to as an elastic material layer). In certain aspects, the elastic layer 106 can be configured to apply force to the underlying layers 102 and 104 in a radial direction (e.g., toward the central axis 114 of the sheath) when the sheath expands beyond its natural diameter by passage of the delivery apparatus through the sheath. Stated differently, the elastic layer 106 can be configured to apply encircling pressure to the layers of the sheath beneath the elastic layer 106 to counteract expansion of the sheath. The radially inwardly directed force is sufficient to cause the sheath to collapse radially back to its unexpanded state after the delivery apparatus is passed through the sheath. It is understood, however, that the elastic layer 106 can be optional. And also described herein are the aspects where this third elastic layer is not present, while all other layers described herein are. It is also understood that this description includes all various combinations of the layers, and unless it is stated otherwise, some of the described herein layers (liners) can be present while others can be absent.

In the illustrated aspect, the elastic layer 106 can comprise one or more members configured as strands, ribbons, or bands 116 helically wrapped around the braided layer 104. For example, in the illustrated aspect the elastic layer 106 comprises two elastic bands 116A and 116B wrapped around the braided layer with opposite helicity, although the elastic layer may comprise any number of bands depending upon the desired characteristics. The elastic bands 116A and 116B can be made from, for example, any of a variety of natural or synthetic elastomers, including silicone rubber, natural rubber, any of various thermoplastic elastomers, polyurethanes such as polyurethane siloxane copolymers, urethane, plasticized polyvinyl chloride (PVC), styrenic block copolymers, polyolefin elastomers, etc. In some aspects, the elastic layer can comprise an elastomeric material having a modulus of elasticity of 200 MPa or less. In some aspects, the elastic layer 106 can comprise a material exhibiting an elongation to break of 200% or greater, or an elongation to break of 400% or greater. The elastic layer 106 can also take other forms, such as a tubular layer comprising an elastomeric material, a mesh, a shrinkable polymer layer such as a heat-shrink tubing layer, etc. In lieu of, or in addition to, the elastic layer 106, the sheath 100 may also include an elastomeric or heat-shrink tubing layer around the outer layer 108. Examples of such elastomeric layers are disclosed in U.S. Publication No. 2014/0379067, U.S. Publication No. 2016/0296730, and U.S. Publication No. 2018/0008407. In other aspects, the elastic layer 106 can also be radially outward of the polymeric layer 108.

In certain aspects, one or both of the inner polymeric layer 102 and/or the outer layer 108 can be configured to resist axial elongation of the sheath 100 when the sheath expands. More particularly, one or both of the inner polymeric layer 102 and/or the outer layer 108 can resist stretching against longitudinal forces caused by friction between a prosthetic device and the inner surface of the sheath such that the length L remains substantially constant as the sheath expands and contracts. As used herein with reference to the length L of the sheath, the term "substantially constant" means that the length L of the sheath increases by not more than 1%, by not more than 5%, by not more than 10%, by not more than 15%, or by not more than 20%. Meanwhile, with reference to FIG. 5B, the filaments 110A and 110B of the braided layer can be allowed to move angularly relative to each other such that the angle θ changes as the sheath expands and contracts. This, in combination with the longitudinally extending folds 126 in the polymeric layers 102 and 108, can allow the central lumen 112 of the sheath to expand as a prosthetic device is advanced through it.

For example, in some aspects the inner polymeric layer 102 and the outer layer 108 can be heat-bonded during the manufacturing process such that the braided layer 104 and the elastic layer 106 are encapsulated between the polymeric layers 102 and 108. More specifically, in certain aspects the inner polymeric layer 102 and the outer polymeric layer 108 can be adhered to each other through the spaces between the filaments 110 of the braided layer 104 and/or the spaces between the elastic bands 116. The polymeric layers 102 and 108 can also be bonded or adhered together at the proximal and/or distal ends of the sheath. In certain aspects, the polymeric layers 102 and 108 are not adhered to the filaments 110. This can allow the filaments 110 to move angularly relative to each other, and relative to the layers 102 and 108, allowing the diameter of the braided layer 104, and thereby the diameter of the sheath, to increase or decrease. As the angle θ between the filaments 110A and 110B changes, the length of the braided layer 104 can also change. For example, as the angle θ increases, the braided layer 104 can foreshorten, and as the angle θ decreases, the braided layer 104 can lengthen to the extent permitted by the areas where the polymeric layers 102 and 108 are bonded. However, because the braided layer 104 is not adhered to the polymeric layers 102 and 108, the change in length of the braided layer that accompanies a change in the angle θ between the filaments 110A and 110B does not result in a significant change in the length L of the sheath.

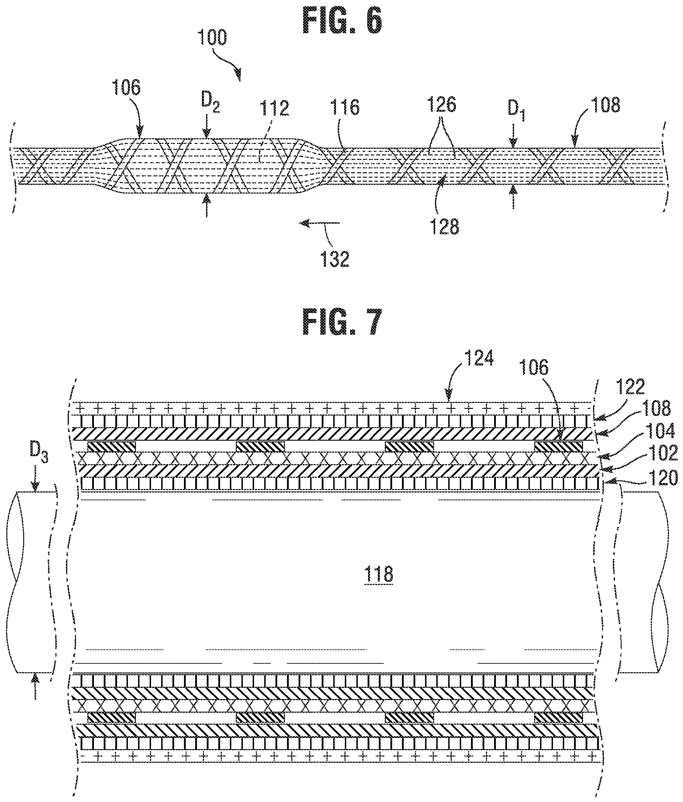

FIG. 6 illustrates a local radial expansion of the sheath 100 as a prosthetic device 12 is passed through the sheath in the direction of arrow 132 (e.g., distally). As the prosthetic device 12 is advanced through the sheath 100, the sheath can resiliently expand to a second diameter D₂ that corresponds to a size or diameter of the prosthetic device. As the prosthetic device 12 is advanced through the sheath 100, the prosthetic device can apply longitudinal force to the sheath in the direction of motion by virtue of the frictional contact between the prosthetic device and the inner surface of the sheath. However, as noted above, the inner polymeric layer 102 and/or the outer polymeric layer 108 can resist axial elongation such that the length L of the sheath remains constant, or substantially constant. This can reduce or prevent the braided layer 104 from lengthening, and thereby constricting the central lumen 112.

Meanwhile, the angle θ between the filaments 110A and 110B can increase as the sheath expands to the second diameter D₂ to accommodate the prosthetic valve. This can cause the braided layer 104 to foreshorten. However, because the filaments 110 are not engaged or adhered to the polymeric layers 102 or 108, the shortening of the braided layer 104 attendant to an increase in the angle θ does not affect the overall length L of the sheath. Moreover, because of the longitudinally extending folds 126 formed in the polymeric layers 102 and 108, the layers 102 and 108 can expand to the second diameter D₂ without rupturing, in spite of being relatively thin and relatively non-elastic. In this manner, the sheath 100 can resiliently expand from its natural diameter D₁ to a second diameter D₂ that is larger than the diameter D₁ as a prosthetic device is advanced through the sheath, without lengthening, and without constricting. Thus, the force required to push the prosthetic implant through the sheath is significantly reduced.

Additionally, because of the radial force applied by the elastic layer 106, the radial expansion of the sheath 100 can be localized to the specific portion of the sheath occupied by the prosthetic device. For example, with reference to FIG. 6, as the prosthetic device 12 moves distally through the sheath 100, the portion of the sheath immediately proximal to the prosthetic device 12 can radially collapse back to the initial diameter D₁ under the influence of the elastic layer 106. The polymeric layers 102 and 108 can also buckle as the circumference of the sheath is reduced, causing the ridges 126 and the valleys 128 to reform. This can reduce the size of the sheath required to introduce a prosthetic device of a given size. Additionally, the temporary, localized nature of the expansion can reduce trauma to the blood vessel into which the sheath is inserted, along with the surrounding tissue, because only the portion of the sheath occupied by the prosthetic device expands beyond the sheath's natural diameter and the sheath collapses back to the initial diameter once the device has passed. This limits the amount of tissue that must be stretched in order to introduce the prosthetic device, and the amount of time for which a given portion of the vessel must be dilated.

In addition to the advantages above, the expandable sheath aspects described herein can provide surprisingly superior performance relative to known introducer sheaths. For example, it is possible to use a sheath configured as described herein to deliver a prosthetic device having a diameter that is two times larger, 2.5 times larger, or even three times larger than the natural outer diameter of the sheath. For example, in one aspect a crimped prosthetic heart valve having a diameter of 7.2 mm was successfully advanced through a sheath configured as described above and having a natural outer diameter of 3.7 mm. As the prosthetic valve was advanced through the sheath, the outer diameter of the portion of the sheath occupied by the prosthetic valve increased to 8 mm. In other words, it was possible to advance a prosthetic device having a diameter more than two times the outer diameter of the sheath through the sheath, during which the outer diameter of the sheath resiliently increased by 216%. In another example, a sheath with an initial or natural outer diameter of 4.5 mm to 5 mm can be configured to expand to an outer diameter of 8 mm to 9 mm.

In alternative aspects, the sheath 100 may optionally include the polymeric layer 102 without the polymeric layer 108, or the polymeric layer 108 without the polymeric layer 102, depending upon the particular characteristics desired.

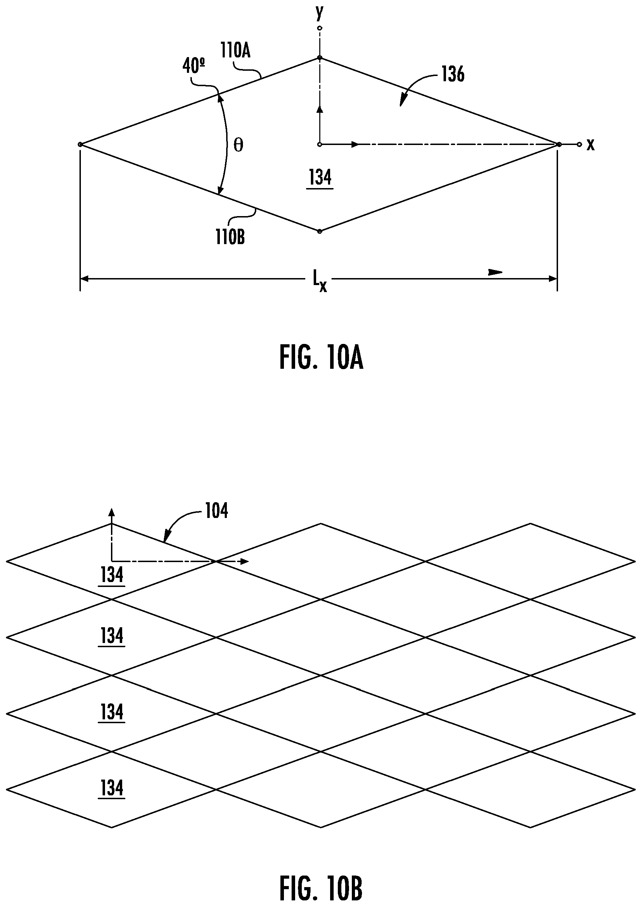

FIGS. 10A-10D illustrate another aspect of the braided layer 104 in which the filaments 110 are configured to buckle. For example, FIG. 10A illustrates a unit cell 134 of the braided layer 104 in a configuration corresponding to the braided layer in a fully expanded state. For example, the expanded state illustrated in FIG. 10A can correspond to the diameter D₂ described above, and/or a diameter of the braided layer during initial construction of the sheath 100 before the sheath is radially collapsed to its functional design diameter D₁, as described further below with reference to FIG. 7. The angle θ between the filaments 110A and 110B can be, for example, 40°, and the unit cell 134 can have a length *Lₓ* along the x-direction (note Cartesian coordinate axes shown). FIG. 10B illustrates a portion of the braided layer 104 including an array of unit cells 134 in the expanded state.

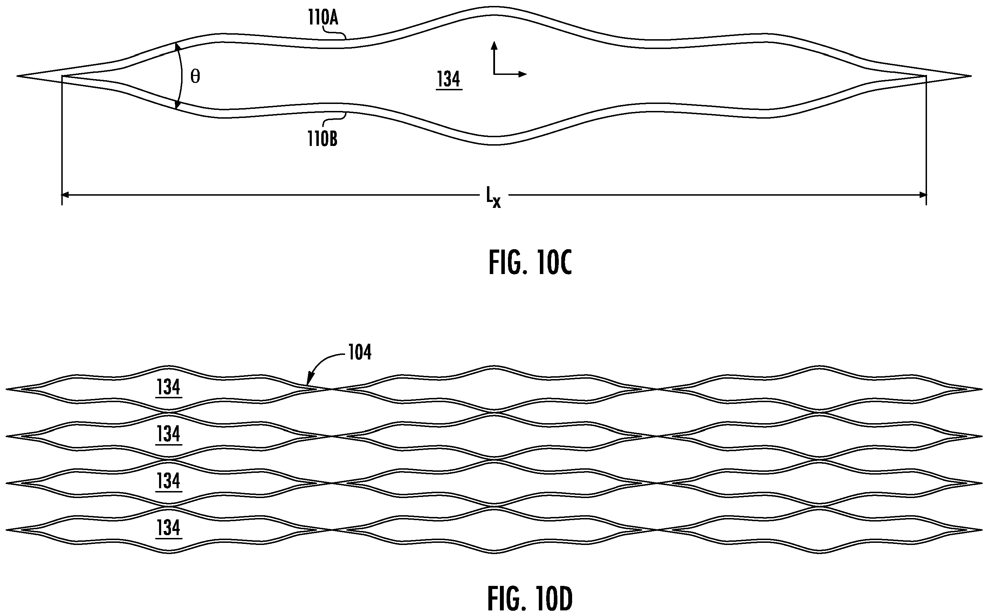

In the illustrated aspects, the braided layer 104 is disposed between the polymeric layers 102 and 108, as described above. For example, the polymeric layers 102 and 108 can be adhered or laminated to each other at the ends of the sheath 100 and/or between the filaments 110 in the open spaces 136 defined by the unit cells 134. Thus, with reference to FIGS. 10C and 10D, when the sheath 100 is radially collapsed to its functional diameter D₁, the diameter of the braided layer 104 can decrease as the angle θ decreases. However, the bonded polymeric layers 102 and 108 can constrain or prevent the braided layer 104 from lengthening as it radially collapses. This can cause the filaments 110 to resiliently buckle in the axial direction, as shown in FIGS. 10C and 10D. The degree of buckling can be such that the length *Lₓ* of the unit cells 134 is the same, or substantially the same, between the collapsed and fully expanded diameters of the sheath. This means that the overall length of the braided layer 104 can remain constant, or substantially constant, between the natural diameter D₁ of the sheath and the expanded diameter D₂. As the sheath expands from in its initial diameter D₁ during passage of a medical device, the filaments 110 can straighten as the buckling is relieved, and the sheath can radially expand. As the medical device passes through the sheath, the braided layer 104 can be urged back to the initial diameter D₁ by the elastic layer 106, and the filaments 110 can resiliently buckle again. Using the configuration of FIGS. 10A-10C, it is also possible to accommodate a prosthetic device having a diameter that is two times larger, 2.5 times larger, or even three times larger than the natural outer diameter D₁ of the sheath.

Turning now to methods of making expandable sheaths, FIG. 7 illustrates the layers 102-108 of the expandable sheath 100 disposed on a cylindrical mandrel 118, according to one aspect. In certain aspects, the mandrel 118 can have a diameter D₃ that is greater than the desired natural outer diameter D₁ of the finished sheath. For example, in some aspects a ratio of the diameter D₃ of the mandrel to the outer diameter D₁ of the sheath can be 1.5:1, 2:1, 2.5:1, 3:1, or greater. In certain aspects, the diameter D₃ of the mandrel can be equal to the expanded diameter D₂ of the sheath. In other words, the diameter D₃ of the mandrel can be the same, or nearly the same, as the desired expanded diameter D₂ of the sheath when a prosthetic device is being advanced through the sheath. Thus, in certain aspects a ratio of the expanded outer diameter D₂ of the expanded sheath to the collapsed outer diameter D₁ of the unexpanded sheath can be 1.5:1, 2:1, 2.5:1, 3:1, or greater.

With reference to FIG. 7, the expandable sheath 100 can be made by wrapping or situating an ePTFE layer 120 around the mandrel 118, followed by the first polymeric layer 102. In some aspects, the ePTFE layer can aid in removing the sheath 100 from the mandrel 118 upon completion of the fabrication process. The first polymeric layer 102 may be in the form of a pre-fabricated sheet that is applied by being wrapped around the mandrel 118, or may be applied to the mandrel by dip-coating, electro-spinning, etc. The braided layer 104 can be situated around the first polymeric layer 102, followed by the elastic layer 106. In aspects in which the elastic layer 106 comprises one or more elastic bands 116, the bands 116 can be helically wrapped around the braided layer 104. In other aspects, the elastic layer 106 may be dip-coated, electro-spun, etc. The outer polymeric layer 108 can then be wrapped, situated, or applied around the elastic layer 106, followed by another layer 122 of ePTFE and one or more layers 124 of heat-shrink tubing or heat-shrink tape.

In particular aspects, the elastic bands 116 can be applied to the braided layer 104 in a stretched, taut, or extended condition. For example, in certain aspects the bands 116 can be applied to the braided layer 104 stretched to a length that is twice their natural, relaxed length. This will cause the completed sheath to radially collapse under the influence of the elastic layer when removed from the mandrel, which can cause corresponding relaxation of the elastic layer, as described below. In other aspects, the first polymeric layer 102 and the braided layer 104 can be removed from the mandrel, the elastic layer 106 can be applied in a relaxed state or moderately stretched state, and then the assembly can be placed back on the mandrel such that the elastic layer is radially expanded and stretched to a taut condition prior to application of the outer polymeric layer 108.

The assembly can then be heated to a sufficiently high temperature that the heat-shrink layer 124 shrinks and compresses the layers 102-108 together. In certain aspects, the assembly can be heated to a sufficiently high temperature such that the inner and outer polymeric layers 102 and 108 become soft and tacky, and bond to each other in the open spaces between the braided layer 104 and the elastic layer 106 and encapsulate the braided layer and the elastic layer. In other aspects, the inner and outer polymeric layers 102, 108 can be reflowed or melted such that they flow around and through the braided layer 104 and the elastic layer 106. In an exemplary aspect, the assembly can be heated at 150°C for 20-30 minutes.

After heating, the sheath 100 can be removed from the mandrel 118, and the heat-shrink tubing 124 and the ePTFE layers 120 and 122 can be removed. Upon being removed from the mandrel 118, the sheath 100 can at least partially radially collapse to the natural design diameter D₁ under the influence of the elastic layer 106. In certain aspects, the sheath can be radially collapsed to the design diameter with the optional aid of a crimping mechanism. The attendant reduction in circumference can buckle the filaments 110 as shown in FIGS. 10C and 10D, along with the inner and outer polymeric layers 102 and 108 to create the longitudinally extending folds 126.

In certain aspects, a layer of PTFE can be interposed between the ePTFE layer 120 and the inner polymeric layer 102, and/or between the outer polymeric layer 108 and the ePTFE layer 122, in order to facilitate separation of the inner and outer polymeric layers 102, 108 from the respective ePTFE layers 120 and 122. In further aspects, one of the inner polymeric layer 102 or the outer polymeric layer 108 may be omitted, as described above.

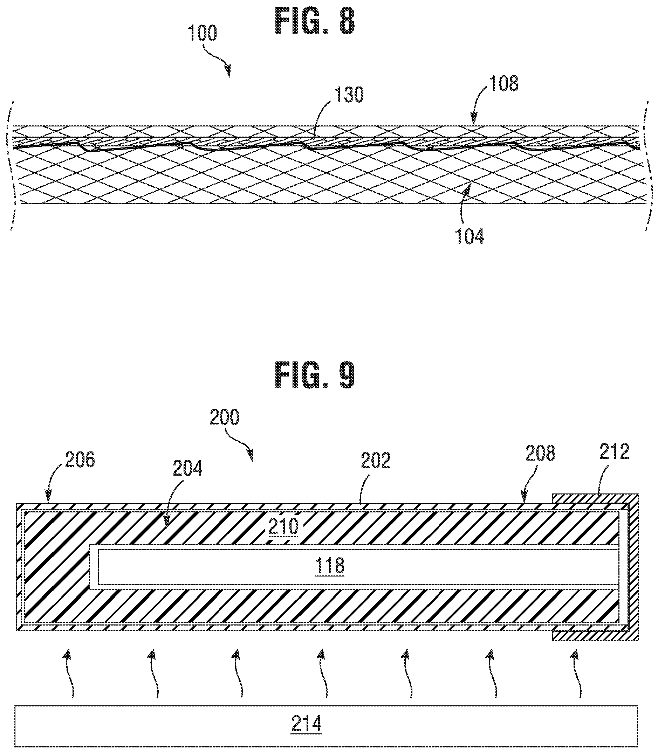

FIG. 8 illustrates another aspect of the expandable sheath 100 including one or more members configured as yarns or cords 130 extending longitudinally along the sheath and attached to the braided layer 104. Although only one cord 130 is illustrated in FIG. 8, in practice the sheath may include two cords, four cords, six cords, etc., arrayed around the circumference of the sheath at equal angular spacings. The cords 130 can be sutured to the exterior of the braided layer 104, although other configurations and attachment methods are possible. By virtue of being attached to the braided layer 104, the cords 130 can be configured to prevent axial elongation of the braided layer 104 when a prosthetic device is passed through the sheath. The cords 130 may be employed in combination with the elastic layer 106, or separately. The cords 130 may also be used in combination with one or both of the inner and/or outer polymeric layers 102 and 108, depending upon the particular characteristics desired. The cords 130 may also be disposed on the inside of the braided layer 104 (e.g., between the inner polymeric layer 102 and the braided layer 104).

The expandable sheath 100 can also be made in other ways. For example, FIG. 9 illustrates an apparatus 200 including a containment vessel 202 and a heating system schematically illustrated at 214. The apparatus 200 is particularly suited for forming devices (medical devices or devices for non-medical uses) comprised of two or more layers of material. Devices formed by the apparatus 200 can be formed from two or more co-axial layers of material, such as the sheath 100, or shafts for catheters. Devices formed by the apparatus 200 alternatively can be formed by two or more non-coaxial layers, such as two or more layers stacked on top of each other.

The containment vessel 202 can define an interior volume or chamber 204. In the illustrated aspect, the vessel 202 can be a metal tube including a closed end 206 and an open end 208. The vessel 202 can be at least partially filled with a thermally-expandable material 210 having a relatively high coefficient of thermal expansion. In particular aspects, the thermally-expandable material 210 may have a coefficient of thermal expansion of 2.4 x 10⁻⁴/°C or greater. Exemplary thermally-expandable materials include elastomers such as silicones materials. Silicone materials can have a coefficient of thermal expansion of from 5.9 x 10⁻⁴/°C to 7.9 x 10⁻⁴/°C.

A mandrel similar to the mandrel 118 of FIG. 7 and including the desired combination of sheath material layers disposed around it can be inserted into the thermally-expandable material 210. Alternatively, the mandrel 118 can be inserted into the chamber 204, and the remaining volume of the chamber can be filled with the thermally-expandable material 210 so that the mandrel is surrounded by the thermally-expandable material 210. The mandrel 118 is shown schematically for purposes of illustration. As such, the mandrel 118 can be cylindrical as depicted in FIG. 7. Likewise, the inner surface of the thermally-expandable material 210 and the inner surface of the vessel 202 can have a cylindrical shape that corresponds to the shape of the mandrel 118 and the final shape of the sheath 100. To facilitate placement of a cylindrical or rounded mandrel 118, the vessel 202 can comprise two portions that are connected to each other by a hinge to allow the two portions to move between an open configuration for placing the mandrel inside of the vessel and a closed configuration extending around the mandrel. For example, the upper and lower halves of the vessel shown in FIG. 9 can be connected to each other by a hinge at the closed side of the vessel (the left side of the vessel in FIG. 9).

The open end 208 of the vessel 202 can be closed with a cap 212. The vessel 202 can then be heated by the heating system 214. Heating by the heating system 214 can cause the thermally-expandable material 210 to expand within the chamber 204 and apply radial pressure against the layers of material on the mandrel 118. The combination of the heat and pressure can cause the layers on the mandrel 118 to bond or adhere to each other to form a sheath. In certain aspects, it is possible to apply radial pressure of 100 MPa or more to the mandrel 118 using the apparatus 200. The amount of radial force applied to the mandrel can be controlled by, for example, the type and quantity of the thermally-expandable material 210 selected and its coefficient of thermal expansion, the thickness of the thermally-expandable material 210 surrounding the mandrel 118, the temperature to which the thermally-expandable material 210 is heated, etc.

In some aspects, the heating system 214 can be an oven into which the vessel 202 is placed. In some aspects, the heating system can include one or more heating elements positioned around the vessel 202. In some aspects, the vessel 202 can be an electrical resistance heating element or an induction heating element controlled by the heating system 214. In some aspects, heating elements can be embedded in the thermally-expandable material 210. In some aspects, the material 210 can be configured as a heating element by, for example, adding electrically conductive filler materials, such as carbon fibers or metal particles.

The apparatus 200 can provide several advantages over known methods of sheath fabrication, including uniform, highly controllable application of radial force to the mandrel 118 along its length, and high repeatability. The apparatus 200 can also facilitate fast and accurate heating of the thermally-expandable material 210, and can reduce or eliminate the need for heat-shrink tubing and/or tape, reducing material costs and labor. The amount of radial force applied can also be varied along the length of the mandrel by, for example, varying the type or thickness of the surrounding thermally-expandable material 210. In certain aspects, multiple vessels 202 can be processed in a single fixture, and/or multiple sheaths can be processed within a single vessel 202. The apparatus 200 can also be used to produce other devices, such as shafts or catheters.

In one specific method, the sheath 100 can be formed by placing layers 102, 104, 106, 108 on the mandrel 118 and placing the mandrel with the layers inside of the vessel 202 with the thermally-expandable material 210 surrounding the outermost layer 108. If desired, one or more inner layers 120 of ePTFE (or similar material) and one or more outer layers 122 of ePTFE (or similar material) can be used (as shown in FIG. 7) to facilitate removal of the finished sheath from the mandrel 118 and the thermally-expandable material 210. The assembly is then heated with the heating system 214 to reflow the polymeric layers 102, 108. Upon subsequent cooling, the polymeric layers 102, 108 become at least partially bonded to each other and at least partially encapsulate layers 104, 106.

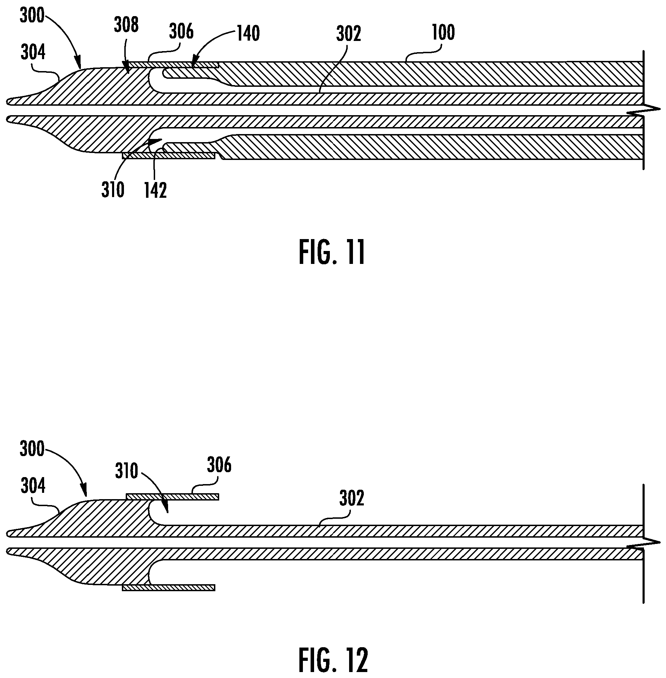

FIG. 11 illustrates another aspect in which the expandable sheath 100 is configured to receive an apparatus configured as a pre-introducer or vessel dilator 300. In particular aspects, the introducer device 90 can include the vessel dilator 300. Referring to FIG. 12, the vessel dilator 300 can comprise a shaft member 302 including a tapered dilator member configured as a nose cone 304 located at the distal end portion of the shaft member 302. The vessel dilator 300 can further comprise a capsule or retaining member 306 extending proximally from a proximal end portion 308 of the nose cone 304 such that a circumferential space 310 is defined between the exterior surface of the shaft member 302 and the interior surface of the retaining member 306. In certain aspects, the retaining member 306 can be configured as a thin polymeric layer or sheet, as further described below.

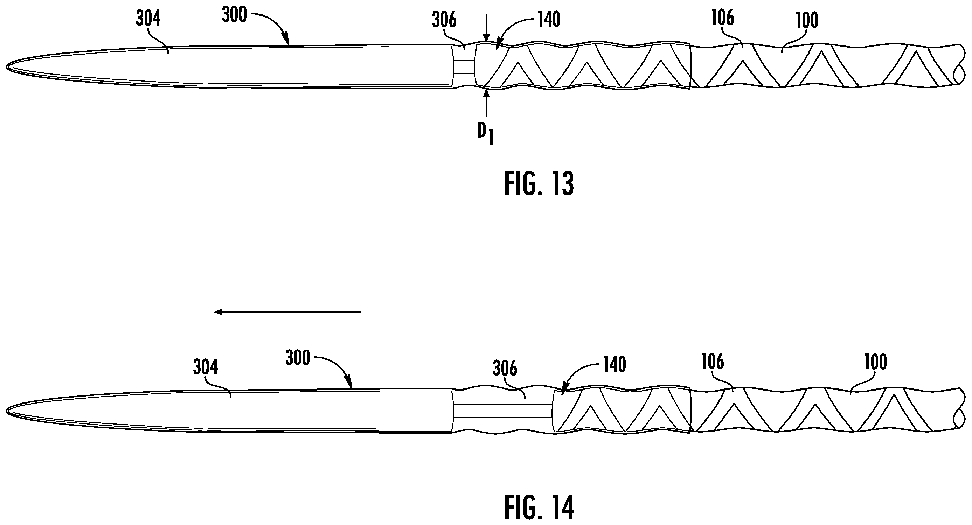

Referring to FIGS. 11 and 13, a first or distal end portion 140 of the sheath 100 can be received in the circumferential space 310 such that the sheath engages the nose cone 304, and/or such that the retaining member 306 extends over the distal end portion 140 of the sheath. In use, the coupled or assembled vessel dilator 300 and sheath 100 can then be inserted through an incision into a blood vessel. The tapered cone shape of the nose cone 304 can aid in gradually dilating the blood vessel and access site while minimizing trauma to the blood vessel and surrounding tissue. Once the assembly has been inserted to the desired depth, the vessel dilator 300 can be advanced further into the blood vessel (e.g., distally) while the sheath 100 is held steady, as illustrated in FIG. 14.

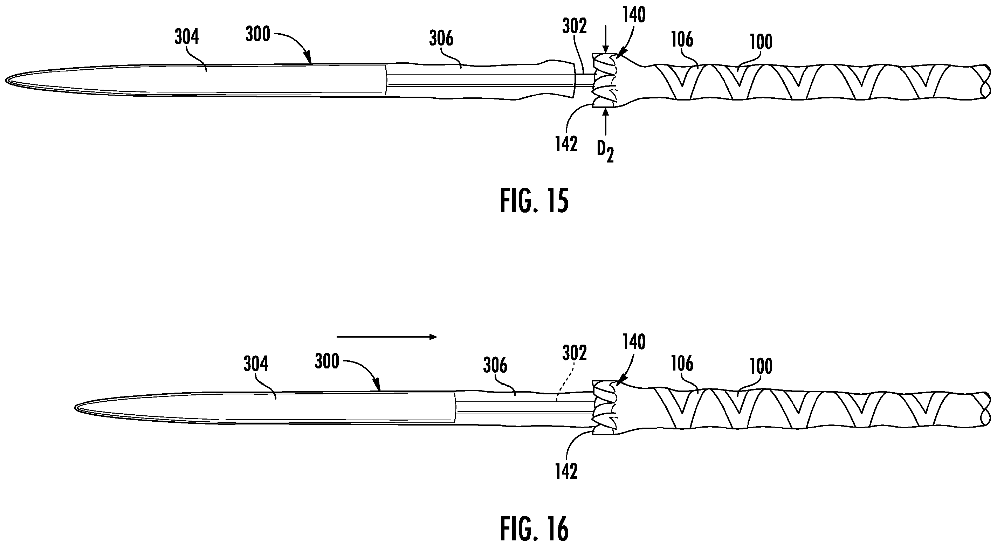

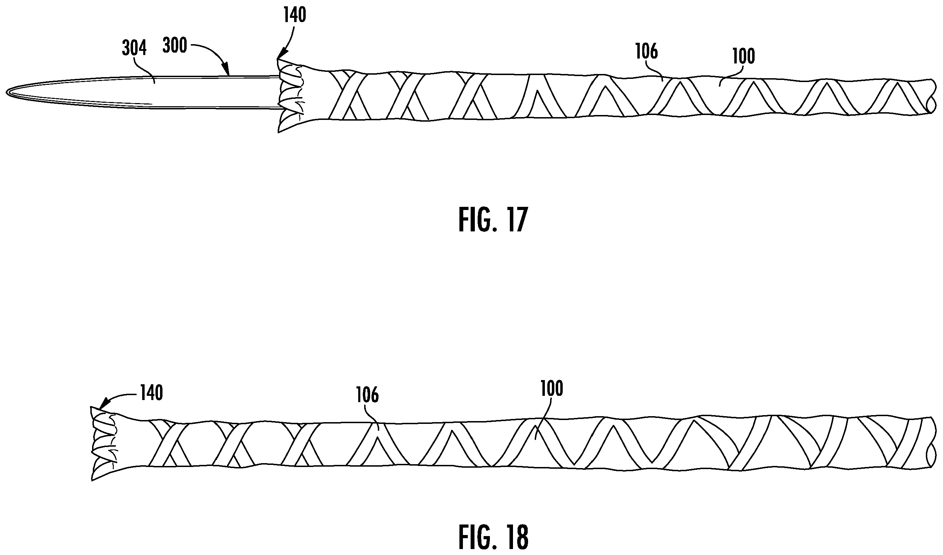

Referring to FIG. 15, the vessel dilator 300 can be advanced distally through the sheath 100 until the retaining member 306 is removed from over the distal end portion 140 of the sheath 100. In certain aspects, the helically-wrapped elastic layer 106 of the sheath can terminate proximally of the distal end 142 of the sheath. Thus, when the distal end portion 140 of the sheath is uncovered, the distal end portion (which can be heat-set) can flare or expand, increasing the diameter of the opening at the distal end 142 from the first diameter D₁ (FIG. 13) to a second, larger diameter D₂ (FIG. 15). The vessel dilator 300 can then be withdrawn through the sheath 100 as illustrated in FIGS. 16-18, leaving the sheath 100 in place in the vessel.

The vessel dilator 300 can include a variety of active and/or passive mechanisms for engaging and retaining the sheath 100. For example, in certain aspects the retaining member 306 can comprise a polymeric heat-shrink layer that can be collapsed around the distal end portion of the sheath 100. In the aspect illustrated in FIG. 1, the retaining member can comprise an elastic member configured to compress the distal end portion 140 of the sheath 100. In yet other aspects, the retaining member 306 and the sheath 100 can be glued or fused (e.g., heat-bonded) together in a manner such that application of selected amount of force can break the adhesive bonds between retaining member 306 free from the sheath 100 to allow the vessel dilator to be withdrawn. In some aspects, the end portion of the braided layer 104 can be heat set to flare or expand radially inwardly or outwardly, in order to apply pressure to a corresponding portion of the vessel dilator 300.

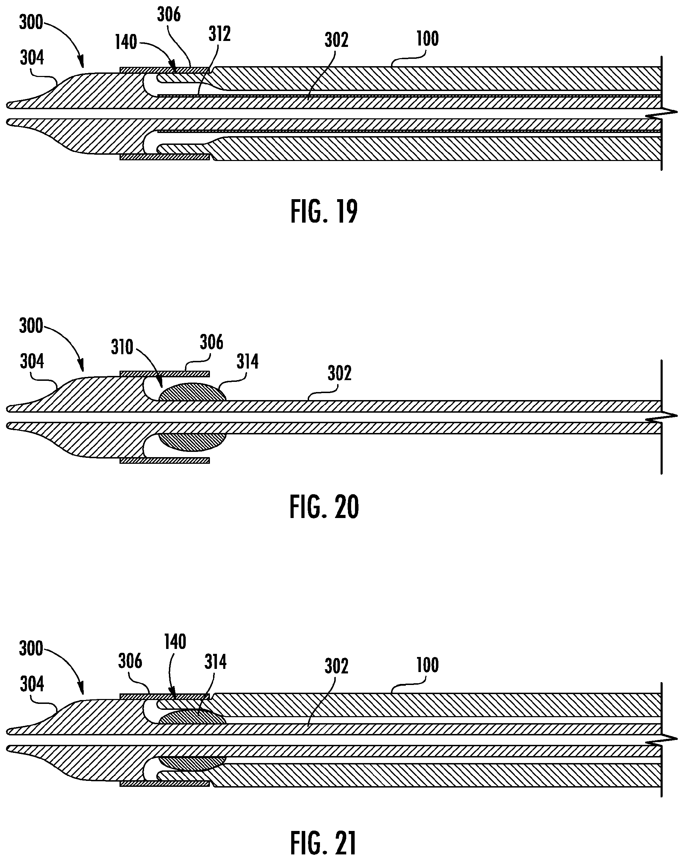

Referring to FIG. 19, the assembly can include a mechanically-actuated retaining mechanism, such as a shaft 312 disposed between the dilator shaft member 302 and the sheath 100. In certain aspects, the shaft 312 can releasably couple the vessel dilator 300 to the sheath 100, and can be actuated from outside the body (i.e., manually deactivated).

Referring to FIGS. 20 and 21, in some aspects the shaft 302 can comprise one or more balloons 314 arrayed circumferentially around its exterior surface and configured to engage the sheath 100 when inflated. The balloons 314 can be selectively deflated in order to release the sheath 100 and withdraw the vessel dilator. For example, when inflated, the balloons press the captured distal end portion of the sheath 100 against the inner surface of the retaining member 306 to assist in retaining the sheath in place relative to the vessel dilator. When the balloons are deflated, the vessel dilator can be more easily moved relative to the sheath 100.

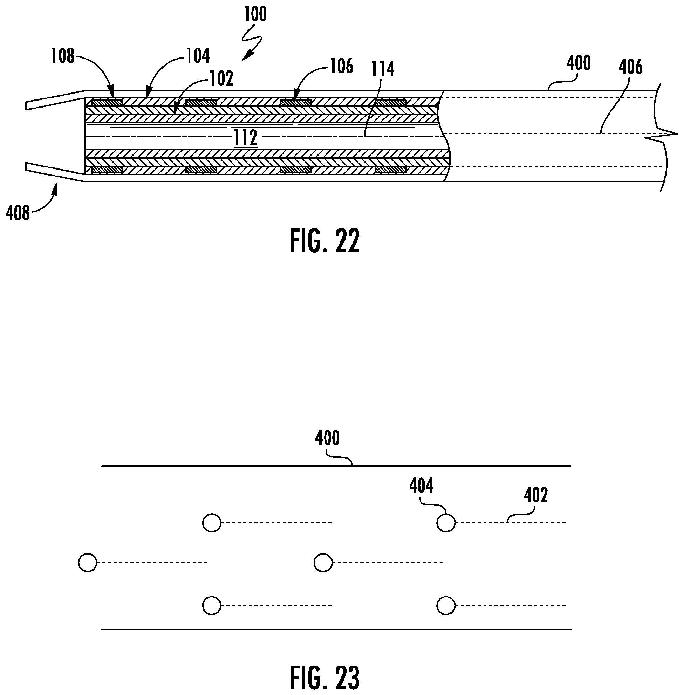

In another aspect, an expandable sheath configured as described above can further comprise a shrinkable polymeric outer cover, such as a heat-shrink tubing layer 400 shown in FIG. 22. The heat-shrink tubing layer 400 can be configured to allow a smooth transition between the vessel dilator 300 and the distal end portion 140 of the sheath. The heat-shrink tubing layer 400 can also constrain the sheath to a selected initial, reduced outer diameter. In certain aspects, the heat-shrink tubing layer 400 extends fully over the length of the sheath 100 and can be attached to the sheath handle by a mechanical fixation means, such as a clamp, nut, adhesive, heat welding, laser welding, or an elastic clamp. In some aspects, the sheath is press-fit into the heat-shrink tubing layer during manufacturing.

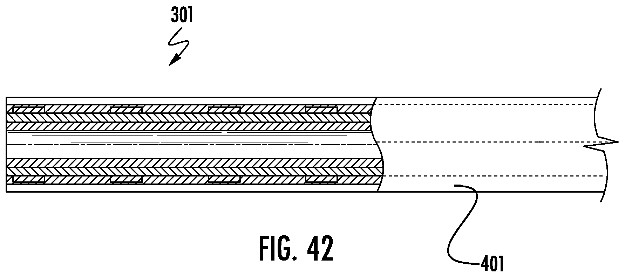

In some aspects, the heat-shrink tubing layer 400 can extend distally beyond the distal end portion 140 of the sheath as the distal overhang 408 shown in FIG. 22. A vessel dilator can be inserted through the central lumen 112 and beyond the distal edge of the overhang 408. The overhang 408 conforms tightly to the inserted vessel dilator to give a smooth transition between the dilator diameter and the sheath diameter to ease insertion of the combined dilator and sheath. When the vessel dilator is removed, overhang 408 remains in the vessel as part of sheath 100. The heat-shrink tubing layer 400 offers the additional benefit of shrinking the overall outer diameter of the sheath along the longitudinal axis. However, it will be understood that some aspects, such as sheath 301 shown at FIG. 42 may have a heat-shrink tubing layer 401 that stops at the distal end of the sheath 301 or, in some aspects, does not extend fully to the distal end of the sheath. In aspects without distal overhangs, the heat-shrink tubing layer functions mainly as an outer shrinking layer, configured to maintain the sheath in a compressed configuration. Such aspects will not result in a flapping overhang at the distal end of the sheath once the dilator is retrieved.

In some aspects, the heat-shrink tubing layer can be configured to split open as a delivery apparatus such as the delivery apparatus 10 is advanced through the sheath. For example, in certain aspects, the heat-shrink tubing layer can comprise one or more longitudinally extending openings, slits, or weakened, elongated scorelines 406 such as those shown in FIG. 22 configured to initiate splitting of the layer at a selected location. As the delivery apparatus 10 is advanced through the sheath, the heat-shrink tubing layer 400 can continue to split open, allowing the sheath to expand as described above with reduced force. In certain aspects, the sheath need not comprise the elastic layer 106 such that the sheath automatically expands from the initial, reduce diameter when the heat-shrink tubing layer splits open. The heat-shrink tubing layer 400 can comprise polyethylene or other suitable materials.

FIG. 23 illustrates a heat-shrink tubing layer 400 that can be placed around the expandable sheaths described herein, according to one aspect. In some aspects, the heat-shrink tubing layer 400 can comprise a plurality of cuts or scorelines 402 extending axially along the heat-shrink tubing layer 400 and terminating at distal stress relief features configured as openings 404. It is contemplated that the distal stress relief feature can be configured as any other regular or irregular curvilinear shape including, for example, oval and/or ovoid shaped openings. It is also contemplated various shaped distal stress relief features along and around the heat-shrink tubing layer 400. As the delivery apparatus 10 is advanced through the sheath, the heat-shrink tubing layer 400 can split open along the scorelines 402, and the distally positioned openings 404 can arrest further tearing or splitting of the tubing layer along the respective scorelines. As such, the heat-shrink tubing layer 400 remains attached to the sheath along the sheath length. In the illustrated aspect, the scorelines and associated openings 404 are longitudinally and circumferentially offset from one another or staggered. Thus, as the sheath expands, the scorelines 402 can form rhomboid structures. The scorelines can also extend in other directions, such as helically around the longitudinal axis of the sheath, or in a zig-zag pattern

In other aspects, splitting or tearing of the heat-shrink tubing layer may be induced in a variety of other ways, such as by forming weakened areas on the tubing surface by, for example, applying chemical solvents, cutting, scoring, or ablating the surface with an instrument or laser, and/or by decreasing the wall thickness or making cavities in the tubing wall (e.g., by femto-second laser ablation).

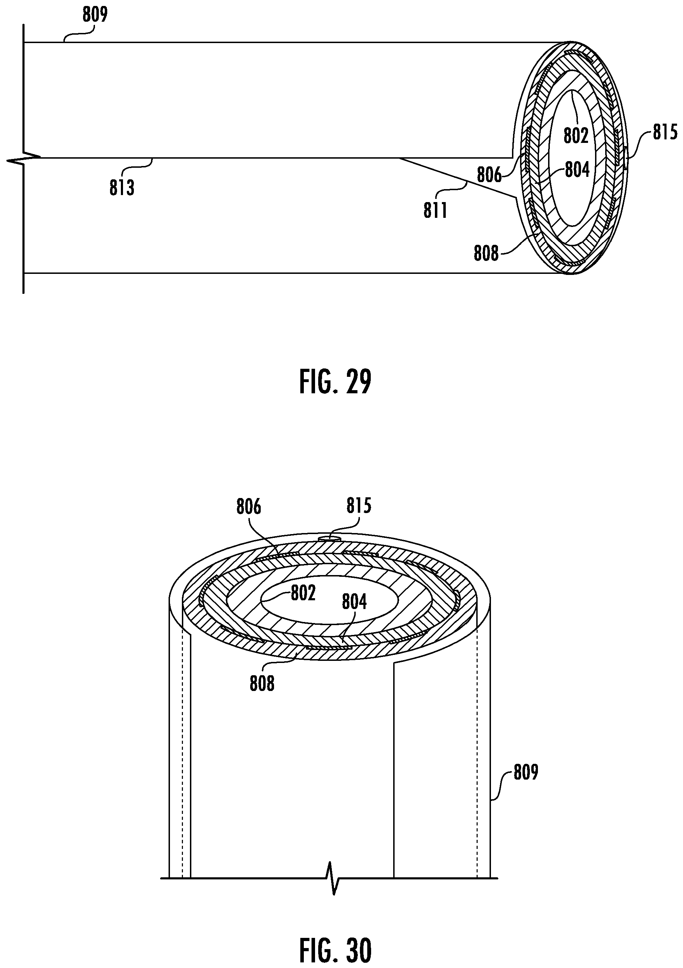

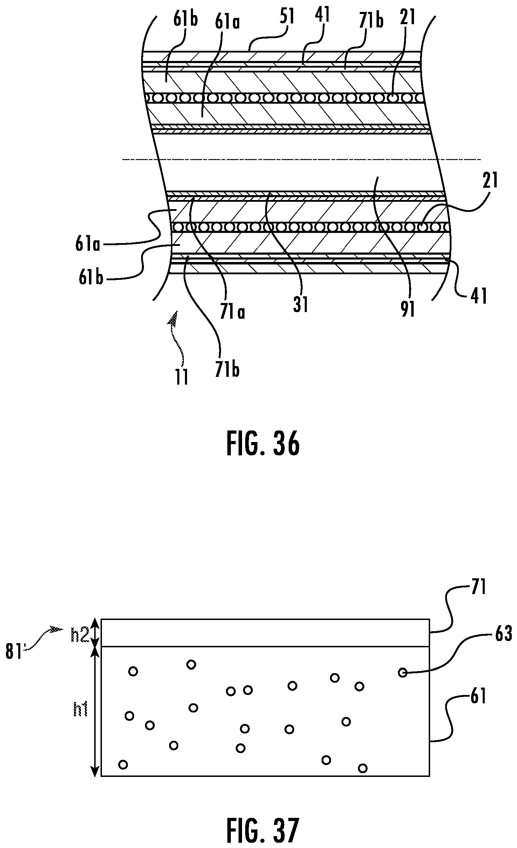

In some aspects, the heat-shrink tubing layer may be attached to the body of the sheath by adhesive, welding, or any other suitable fixation means. FIG. 29 shows a perspective view of a sheath aspect including an inner layer 802, a braided layer 804, an elastic layer 806, an outer layer 808, and a heat shrink tubing layer 809. As described below with respect to FIG. 36, some aspects may not include elastic layer 806. Heat shrink tubing layer 809 includes a split 811 and a perforation 813 extending along the heat shrink tubing layer 809. Heat shrink tubing layer 809 is bonded to the outer layer 808 at an adhesive seam 815. For example, in certain aspects the heat-shrink tubing layer 809 can be welded, heat-bonded, chemically bonded, ultrasonically bonded, and/or bonded using adhesive agents (including, but not limited to, hot glue, for example LDPE fiber hot glue) at seam 815. The outer layer 808 can be bonded to the heat shrink tubing layer 809 axially along the sheath at a seam 815, or in a spiral or helical fashion. FIG. 30 shows the same sheath aspect with heat shrink tubing layer 809 split open at the distal end of the sheath.

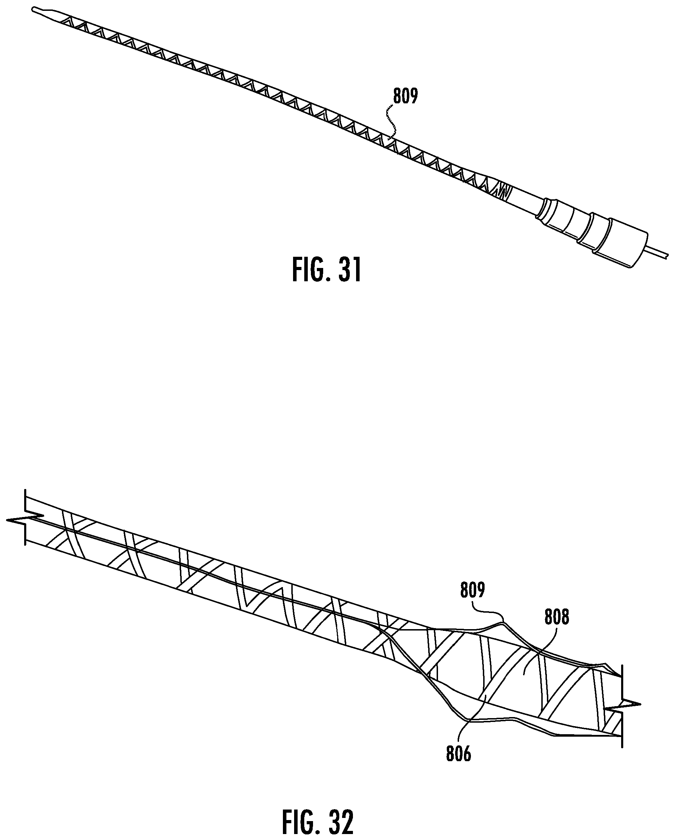

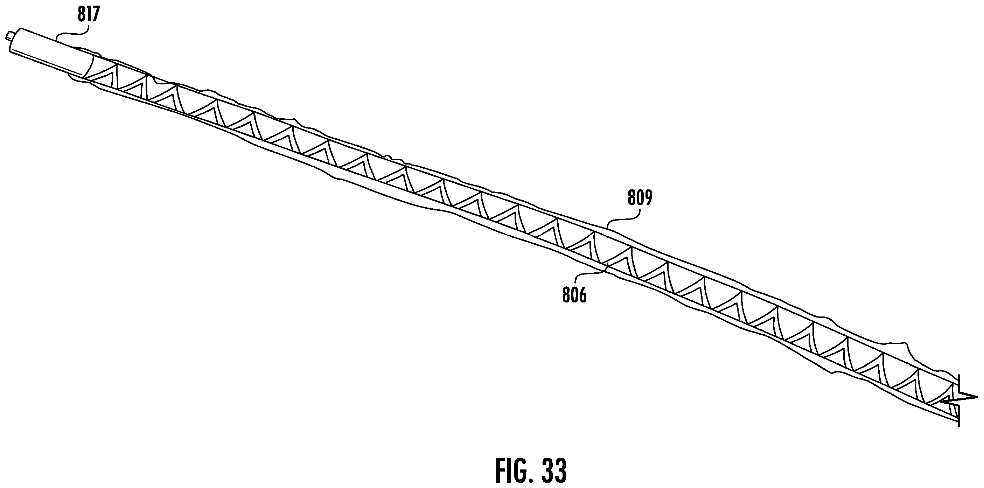

FIG. 31 shows a sheath having a heat shrink tubing layer 809, but prior to movement of a delivery system therethrough. FIG. 32 shows a perspective view of a sheath wherein the heat shrink tubing layer 809 has been partially torn open and detached as a passing delivery system widens the diameter of the sheath. Heat shrink tubing layer 809 is being retained by the adhesive seam 815. Attaching the heat-shrink tubing layer 809 to the sheath in this manner can help to keep the heat-shrink tubing layer 809 attached to the sheath after the layer splits and the sheath has expanded, as shown in FIG. 33, where delivery system 817 has moved completely through the sheath and torn the heat shrink tubing layer 809 along the entire length of the sheath.

In another aspect, the expandable sheath can have a distal end or tip portion comprising an elastic thermoplastic material (e.g., Pebax), which can be configured to provide an interference fit or interference geometry with the corresponding portion of the vessel dilator 300. In certain configurations, the outer layer of the sheath may comprise polyamide (e.g., nylon) in order to provide for welding the distal end portion to the body of the sheath. In certain aspects, the distal end portion can comprise a deliberately weakened portion, scoreline, slit, etc., to allow the distal end portion to split apart as the delivery apparatus is advanced through the distal end portion.

In another aspects, the entire sheath could have an elastomeric outer cover that extends longitudinally from the handle to the distal end portion 140 of the sheath, optionally extending onward to create an overhang similar to overhang 408 shown in FIG. 22. The elastomeric overhang portion conforms tightly to the vessel dilator but remains a part of the sheath once the vessel dilator is removed. As a delivery system is passed through, the elastomeric overhang portion expands and then collapses to allow it to pass. The elastomeric overhang portion, or the entire elastomeric outer cover, can include deliberately weakened portions, scorelines, slits, etc. to allow the distal end portion to split apart as the delivery apparatus is advanced through the distal end portion.

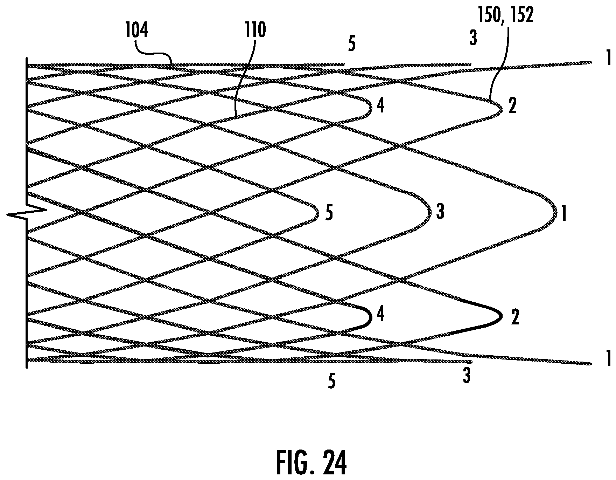

FIG. 24 illustrates an end portion (e.g., a distal end portion) of another aspect of the braided layer 104 in which portions 150 of the filaments 110 are bent to form loops 152, such that the filaments loop or extend back in the opposite direction along the sheath. The filaments 110 can be arranged such that the loops 152 of various filaments 110 are axially offset from each other in the braid. Moving toward the distal end of the braided layer 104 (to the right in the figure), the number of filaments 110 can decrease. For example, the filaments indicated at 5 can form loops 152 first, followed by the filaments indicated at 4, 3, and 2, with the filaments at 1 forming the distal-most loops 152. Thus, the number of filaments 110 in the braid decreases in the distal direction, which can increase the radial flexibility of the braided layer 104.

In another aspect, the distal end portion of the expandable sheath can comprise a polymer such as Dyneema^{®}, which can be tapered to the diameter of the vessel dilator 300. Weakened portions such as dashed cuts, scoring, etc., can be applied to the distal end portion such that it will split open and/or expand in a repeatable way.

Crimping of the expandable sheath aspects described herein can be performed in a variety of ways, as described above. In additional aspects, the sheath can be crimped using a conventional short crimper several times longitudinally along the longer sheath. In other aspects, the sheath may be collapsed to a specified crimped diameter in one or a series of stages in which the sheath is wrapped in heat-shrink tubing and collapsed under heating. For example, a first heat shrink tube can be applied to the outer surface of the sheath, the sheath can be compressed to an intermediate diameter by shrinking the first heat shrink tube (via heat), the first heat shrink tube can be removed, a second heat shrink tube can be applied to the outer surface of the sheath, the second heat shrink tube can be compressed via heat to a diameter smaller than the intermediate diameter, and the second heat shrink tube can be removed. This can go on for as many rounds as necessary to achieve the desired crimped sheath diameter.

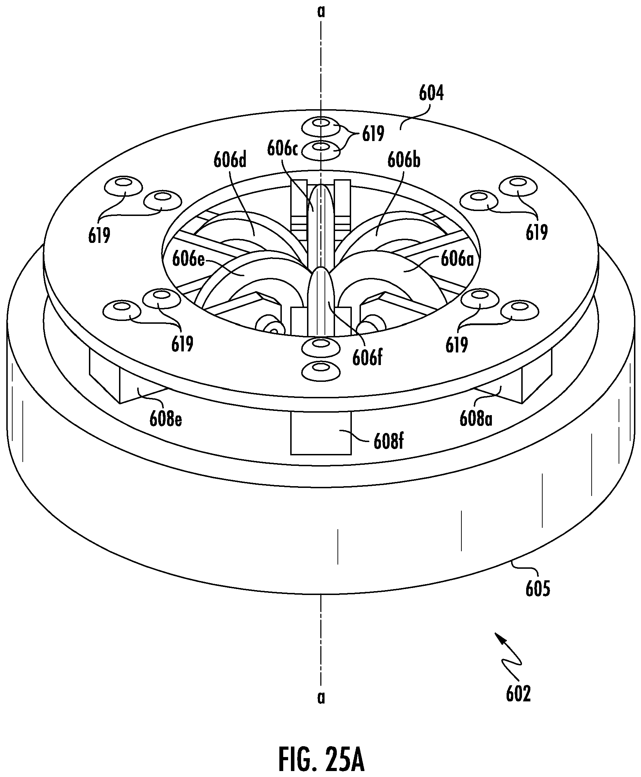

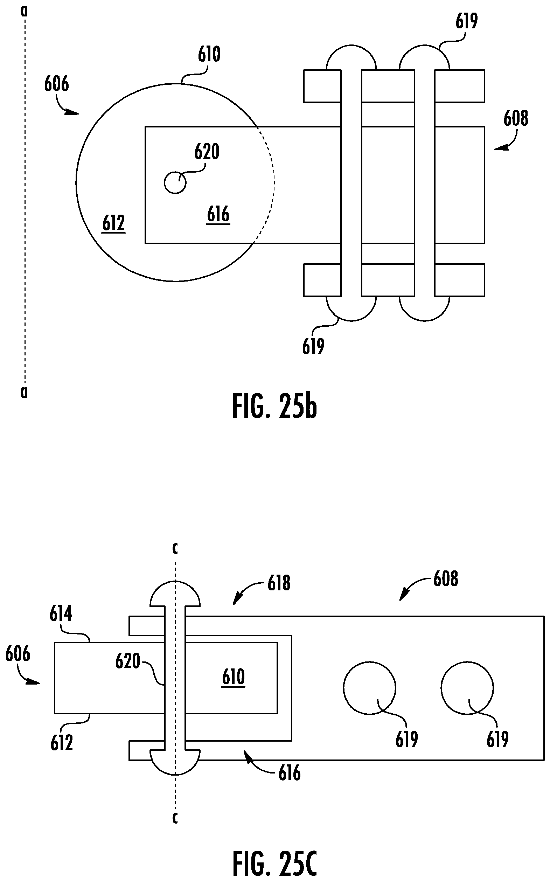

Crimping of the expandable sheath aspects described herein can be performed in a variety of ways, as described above. A roller-based crimping mechanism 602, such as the one shown in FIGS. 25A-25C, can be advantageous for crimping elongated structures such as the sheaths disclosed herein. The crimping mechanism 602 has a first end surface 604, a second end surface 605, and a longitudinal axis *a-a* extending between the first and second end surfaces 604, 605. A plurality of disc-shaped rollers 606a-f are radially arranged about the longitudinal axis *a-a,* each positioned at least partially between the first and second end surfaces of the crimping mechanism 602. Six rollers are depicted in the aspect shown, but the number of rollers may vary. Each disc-shaped roller 606 is attached to the larger crimping mechanism by a connector 608. A side cross-sectional view of an individual disc-shaped roller 606 and connector 608 is shown in FIG. 25B, and a top view of an individual disc-shaped roller 606 and connector 608 is shown in FIG. 25C. An individual disc-shaped roller 606 has a circular edge 610, a first side surface 612, a second side surface 614, and a central axis *c-c* extending between center points of first and second side surfaces 612, 614, as shown in FIG. 25C. The plurality of disc-shaped rollers 606a-f are radially arranged about the longitudinal axis *a-a* of the crimping mechanism 602 such that each central axis *c-c* of a disc-shaped roller 606 is oriented perpendicularly to the longitudinal axis *a-a* of the crimping mechanism 602. The circular edges 610 of the disc-shaped rollers partially define a passage that extends axially through the crimping mechanism 602 along longitudinal axis *a-a.*

Each disc-shaped roller 606 is held in place in the radially arranged configuration by a connector 608 that is attached to crimping mechanism 602 via one or more fasteners 619, such that the location of each of the plurality of connectors is fixed with respect to the first end surface of the crimping mechanism 602. In the depicted aspect, fasteners 619 are positioned adjacent an outer portion of the crimping mechanism 602, radially outwardly of the disc-shaped rollers 606. Two fasteners 619 are used to position each connector 608 in the aspect shown, but the number of fasteners 619 can vary. As shown in FIGS. 25B and 25C, a connector 608 has a first arm 616 and a second arm 618. First and second arms 616, 618 extend over a disc-shaped roller 606 from a radially-outward portion of circular edge 610 to a central portion of the disc-shaped roller 606. A bolt 620 extends through the first and second arms 616, 618 and through a central lumen of the disc-shaped roller 606, the central lumen passing from a center point of the first side surface 612 to a center point of the second side surface 614 of the disc-shaped roller 606 along central axis *c-c.* The bolt 620 is positioned loosely within the lumen, with substantial clearance/space to allow the disc-shaped roller 606 to rotate about central axis *c-c.*

During use, an elongated sheath is advanced from the first end surface 604 of the crimping mechanism 602, through the axial passage between the rollers, and out the second end surface 605 of the crimping mechanism 602. The pressure from the circular edge 610 of the disc shaped rollers 606 reduces the diameter of the sheath to a crimped diameter as it rolls along the outer surface of the elongated sheath.

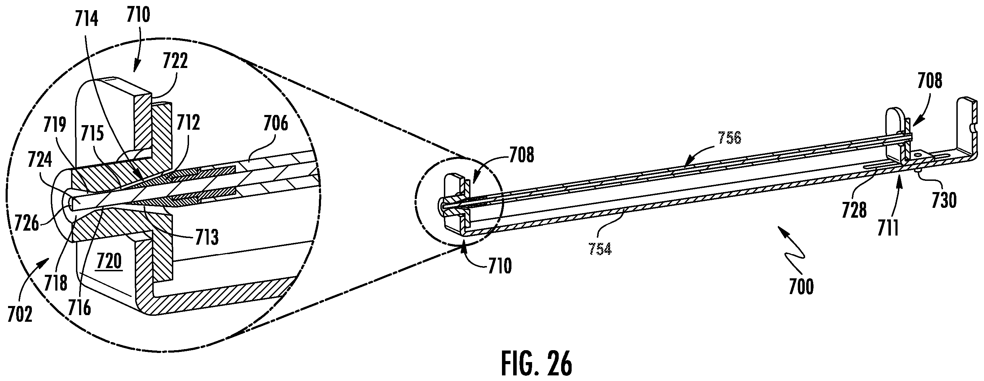

FIG. 26 shows an aspect of a crimping device 700 designed to facilitate crimping of elongated structures, such as sheaths. The crimping device includes an elongated base 754, and elongated mandrel 756 positioned above the elongated base 754, and a holding mechanism 708 attached to the elongated base 754. The holding mechanism 708 supports the mandrel 756 in an elevated position above base 754. The holding mechanism includes a first end piece 710 that includes a crimping mechanism 702. The mandrel 756 includes a conical end portion 712 that nests within a first tapered portion 713 of a narrowing lumen 714 of the first end piece 710. The conical end portion 712 of mandrel 756 is positioned loosely within the narrowing lumen 714, with enough space or clearance between the conical end portion 712 and the lumen 714 to allow for passage of an elongated sheath over the conical end portion 712 of mandrel 756 and through the narrowing lumen 714. During use, the conical end portion 712 helps to avoid circumferential buckling of the sheath during crimping. In some aspects, the mandrel 756 can also include a cylindrical end portion 724 that extends outwardly from the conical end portion 712 and defines an end 726 of the mandrel 756.

The first tapered portion 713 of the narrowing lumen 714 opens toward a second end piece 711 of the holding mechanism 708, such that the widest side of the taper is located on an inner surface 722 of the first end piece 710. In the aspect shown, the first tapered portion 713 narrows to a narrow end 715 that connects with a narrow cylindrical portion 716 of the narrowing lumen 714. In this aspect, the narrow cylindrical portion 716 defines the narrowest diameter of the narrowing lumen 714. The cylindrical end portion 724 of the mandrel 756 may nest loosely within the narrow cylindrical portion 716 of the narrowing lumen 714, with enough space or clearance between the cylindrical end portion 724 and the narrow cylindrical portion 716 of the lumen to allow for passage of the elongated sheath. The elongated nature of the narrow cylindrical portion 716 may facilitate smoothing of the crimped sheath after it has passed over the conical end portion 712 of the mandrel. However, the length of the cylindrical portion 716 of the narrowing lumen 714 is not meant to limit the disclosure, and in some aspects, the crimping mechanism 702 may only include first tapered portion 713 of the narrowing lumen 714, and still be effective to crimp an elongated sheath.

At the opposite end of the first end piece 710 shown in FIG. 26, a second tapered portion 718 of the narrowing lumen 714 opens up from narrow cylindrical portion 716 such that the widest side of the taper located on the outer surface 720 of the first end piece 710. The narrow end 719 of the second tapered portion 718 connects with the narrow cylindrical portion 716 of the narrowing lumen 714 in the interior of the crimping mechanism 702. The second tapered portion 718 of the narrowing lumen 714 may not be present in some aspects.

The holding mechanism 708 further includes a second end piece 711 positioned opposite the elongated base 754 from the first end piece 710. The second end piece 711 is movable with respect to elongated base 754, such that the distance between the first end piece 710 and the second end piece 711 is adjustable and therefore able to support mandrels of varying sizes. In some aspects, elongated base 754 may include one or more elongated sliding tracks 728. The second end piece 711 can be slidably engaged to the sliding track 728 via at least one reversible fastener 730, such as, but not limited to, a bolt that extends into or through the second end piece 711 and the elongated sliding track 728. To move the second end piece 711, the user would loosen or remove the reversible fastener 730, slide the second end piece 711 to the desired location, and replace or tighten the reversible fastener 730.

In use, a sheath in an uncrimped diameter can be placed over the elongated mandrel 756 of the crimping device 700 shown in FIG. 26, such that the inner surface of the entire length of the uncrimped sheath is supported by the mandrel. The uncrimped sheath is then advanced over the conical end portion 712 and through the narrowing lumen 714 of the crimping mechanism 702. The uncrimped sheath is crimped to a smaller, crimped diameter via pressure from the interior surface of the narrowing lumen 714. In some aspects, the sheath is advanced through both a first tapered portion 713 and a cylindrical portion 716 of the narrowing lumen 714 before exiting the crimping mechanism 702. In some aspects, the sheath is advanced through a first tapered portion 713, a cylindrical portion 716, and a second tapering portion 718 of the narrowing lumen 714 before exiting the crimping mechanism 702.

In some aspects, the crimping mechanism 602 shown in FIG. 25A may be positioned within a larger crimping device such as crimping device 700 shown in FIG. 26. For example, the crimping mechanism 602 can be positioned within the first end piece 710 of crimping device 700 instead of, or in combination with, crimping mechanism 702. For example, the rolling crimping mechanism 602 could entirely replace the narrowing lumen 714 of crimping mechanism 702, or the rolling crimping mechanism 602 could be nested within the narrow cylindrical portion 716 of the narrowing lumen 714 of the crimping mechanism 702, such that the first tapered portion 713 feeds the expandable sheath through the plurality of radially arranged disc-shaped rollers 606.

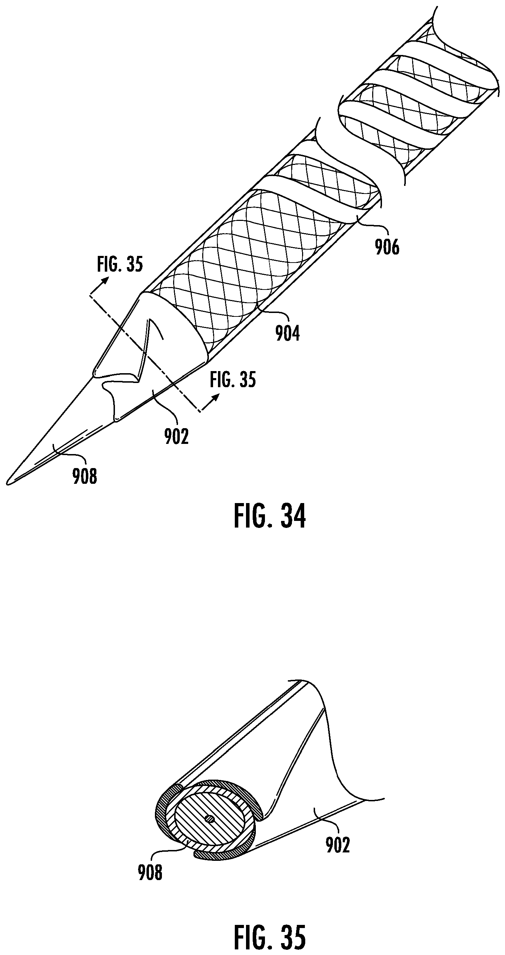

FIGS. 34-35 show a sheath aspect including a distal end portion 902, which can be an extension of an outer cover extending longitudinally along the sheath in the proximal direction. FIG. 34 shows a distal end portion 902 folded around an introducer (in the crimped and collapsed configuration). FIG. 35 shows a cross section of the distal end portion 902 folded around the introducer 908 (in the crimped and collapsed or unexpanded configuration). The distal end portion 902 can be formed of, for example, and without limitation, one or more layers of a similar or the same material used to form the outer layer of the sheath.

However, there are also aspects where the distal end portion of the sheath can include additional materials that are used in addition or instead of the material similar to the one used in the outer layer. For example, and without limitation, the distal portion of the sheath can include layers of materials that exceed the number of layers in other parts of the sheath. In some aspects, the distal end portion 902 includes an extension of the outer layer of the sheath, with or without one more additional layers added by separate processing techniques. The distal end portion can include anywhere from 1 to 8 layers of material (including 1, 2, 3, 4, 5, 6, 7, and 8 layers of material). In some aspects, the distal end portion comprises multiple layers of a Dyneema^{®} material. The distal end portion 902 can extend distally beyond a longitudinal portion of the sheath that includes braided layer 904 and elastic layer 906. In fact, in some aspects, the braided layer 904 may extend distally beyond the elastic layer 906, and the distal end portion 902 may extend distally beyond both the braided layer 904 and elastic layer 906, as shown in FIGS. 34-35.

The distal end portion 902 may have a smaller collapsed diameter than the more proximal portions of the sheath, giving it a tapered appearance. This smooths the transition between the introducer/dilator and the sheath, ensuring that the sheath does not get lodged against the tissue during insertion into the patient. The smaller collapsed diameter can be a result of multiple folds (for example, 1, 2, 3, 4, 5, 6, 7, or 8 folds) positioned circumferentially (evenly or unevenly spaced) around the distal end portion. For example, a circumferential segment of the distal end portion can be brought together and then laid against the adjacent outer surface of the distal end portion to create an overlapping fold. In the collapsed configuration, the overlapping portions of the fold extend longitudinally along the distal end portion 902. Exemplary folding methods and configurations are described in U.S. Application Number 14/880,109 and U.S. Application Number 14/880,111. Scoring can be used as an alternative, or in addition to folding of the distal end portion. Both scoring and folding of the distal end portion 902 allow for the expansion of the distal end portion upon the passage of the delivery system, and ease the retraction of the delivery system back into the sheath once the procedure is complete. In some aspects, the distal end portion of the sheath (and/or of the vessel dilator) can decrease from the initial diameter of the sheath (e.g., 8 mm) to 3.3 mm (10F), and may decrease to the diameter of a guide wire, allowing the sheath and/or the vessel dilator 300 to run on a guide wire.

In some aspects, a distal end portion can be added, the sheath and tip can be crimped, and the crimping of the distal end portion and sheath can be maintained, by the following method. As mentioned above, the distal end portion 902 can be an extension of the outer layer of the sheath. It can also be a separate, multilayer tubing that is heat bonded to the remainder of the sheath prior to the tip crimping processing steps. In some aspects, the separate, multilayer tubing is heat to a distal extension of the outer layer of the sheath to form the distal end portion 902. For crimping of the sheath after tip attachment, the sheath is heated on small mandrel. The distal end portion 902 can be folded around the mandrel to create the folded configuration shown in FIG. 34. The folds be added to the distal end portion 902 prior to the tip crimping process, or at an intermediate point during the tip crimping process. In some aspects, the small mandrel can be from about 2 millimeters to about 4 millimeters in diameter (including about 2.2 millimeters, about 2.4 millimeters, about 2.6 millimeters, about 2.8 millimeters, about 3.0 millimeters, about 3.2 millimeters, about 3.4 millimeters, about 3.6 millimeters, about 3.8 millimeters and about 4.0 millimeters). The heating temperature will be lower than the melting point of the material used. This can cause the material to shrink on its own to a certain extent. For example, in some aspects, such as those where Dyneema^{®} materials are utilized as part of the sheath outer layer and/or distal end portion materials, a sheath crimping process begins by heating the sheath on a 3 millimeter mandrel to about 125 degrees Celsius (lower than Dyneema^{®} melting point of about 140 degrees Celsius). This causes the sheath to crimp itself to about a 6 millimeter outer diameter. At this point, the sheath and distal end portion 902 are allowed to cool. A heat shrink tube can then be applied. In some aspects, the heat shrink tube can have a melting point that is about the same as the melting point of the distal end portion material. The sheath with the heat shrink tube extending over the sheath and the distal end portion 902 is heated again (for example, to about 125 degrees Celsius for sheaths including Dyneema^{®} outer layers and distal end portions), causing the sheath to crimp to an even smaller diameter. At the distal end portion 902, a higher temperature can be applied (for example, from about 145 degrees Celsius to about 155 degrees Celsius for Dyneema^{®} material) causing the layers of material to melt together in the folded configuration shown in FIG. 34 (the folds can be added at any point during this process). The bonds at the distal end portion 902 induced by the high temperature melting step will still be weak enough to be broken by a passing delivery system. As a final step, the heat shrink tube is removed, and the shape of the sheath remains at the crimped diameter.

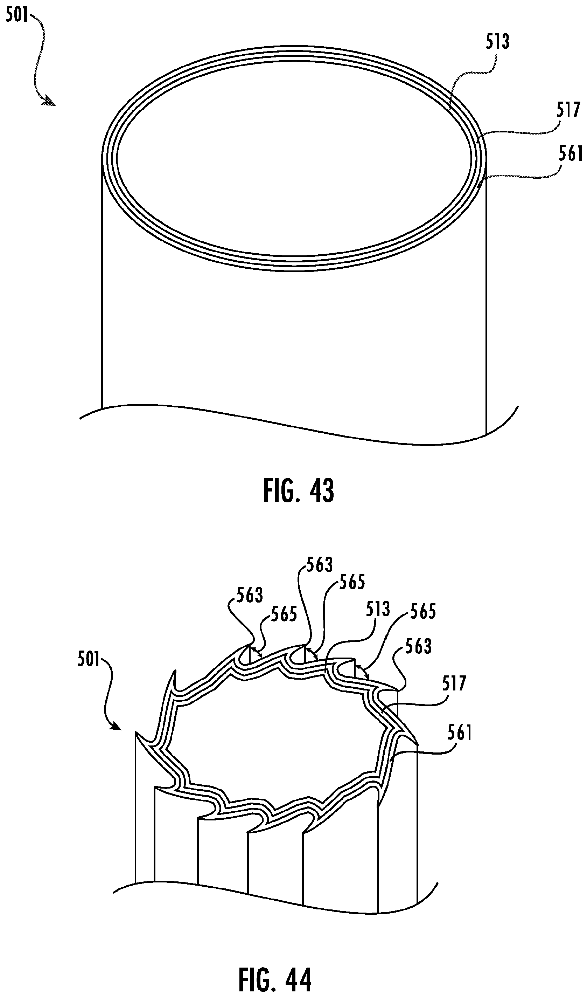

FIG. 43 shows a transverse cross section taken near the distal end of another sheath aspect, at a point longitudinally distal to the braided layer. The sheath 501 includes an inner polymeric layer 513, an outer polymeric layer 517, and an external covering layer 561. A method of compressing the distal portion of an expandable sheath can include: covering at pre-crimped state the distal portion of the expandable sheath 501 with an external covering layer 561 having a melting temperature TM1 which is lower than the melting temperature TM2 of the inner and outer polymeric layers; heating at least one region, which does not span the entire area of overlap between the external covering layer 561 and the expandable sheath 501, to a first temperature which is equal or higher than TM2, thereby melting both the external covering layer 561 and the outer polymeric layer 517 of the expandable sheath 501, so as to create at attachment region 569 there between; inserting a mandrel into the lumen of the expandable sheath 501 and crimping at least a portion thereof, such as the distal portion, of the expandable sheath 501; heating the external covering layer 561 over the distal portion of the expandable sheath 501 to a second temperature which is at least equal to or higher than the melting temperature TM1 of the external covering layer 561, and lower than the melting temperature TM2 of the inner and outer polymeric layers, for a predefined first time window.

This method advantageously avoids risks that a tear initiated at a score or split line (such as perforation 813 shown in FIG. 29) should divert from the intended axial direction of tear propagation due to defects (weakened points or unintended apertures) in the heat-shrink tubing. This method further enables choosing an external covering layer made of materials that may be heated to form moderately attached folds at temperatures lower than those required for the internal or external layers of the expandable sheath.

The crimping of the inner and outer polymeric layers 513, 517 and the external covering layer 561 can be, for example, from a pre-compressed diameter of about 8.3 mm to a compressed diameter of about 3 mm. FIG. 44 shows a transverse cross section of the aspect of FIG. 43 during crimping. Folds 563 are created along the external covering layer 561 during crimping. The heating to the second temperature is sufficient to melt the external covering layer 561 so as to attach the fold 563 to each other, while avoiding similar melting and attachment of the inner and outer polymeric layers.

The method of compressing the distal portion of the expandable sheath can further include a step of covering the expandable sheath 501 and the external covering layer 561 with a heat-shrink tube (HST) prior to, during or following the heating to the second temperature, wherein the second temperature further acts to shrink the HST in order to retain the external covering layer 561 and the expandable sheath 501 in a compressed state. The HST can be removed from the expandable sheath 501 and the external covering layer 561 after the folds 563 of the external covering layer 561 are sufficiently attached to each other in the desired compressed state, and cooled down for a sufficient period of time.

According to some aspects, the HST is further utilized as a heat shrink tape, to apply the external radial pressure by wrapping and heating it over the external covering layer 561 and the expandable sheath 501.

According to some aspects, a non heat-shrink tape can be used instead of a heat shrink tube.

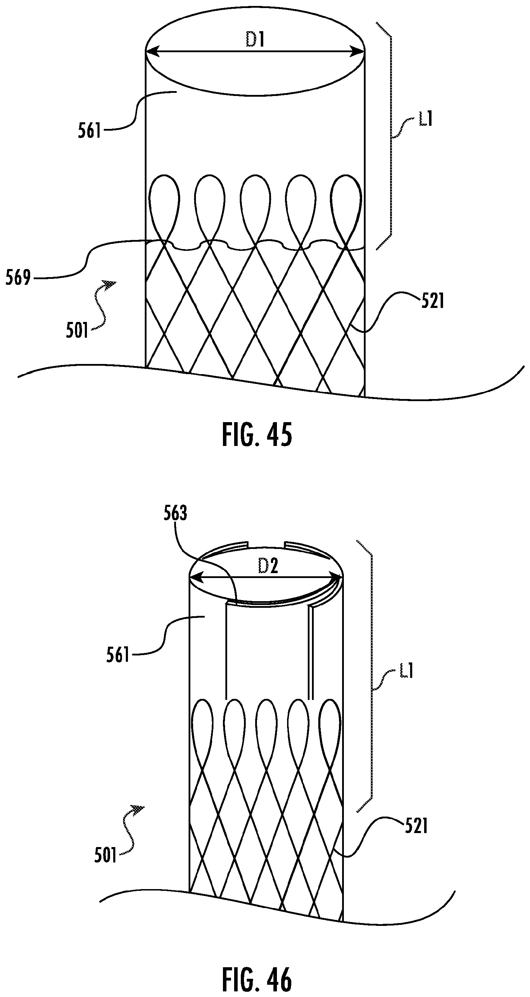

FIG. 45 shows a distal portion of an expandable sheath 501 having an expandable braid 521, wherein its distal portion is covered by an external covering layer 561, which is shown to extend along a length L1 up to the distal edge of the expandable sheath 501. D1 denotes the distal diameter of the expandable sheath 501in the pre-compressed state. Fig. 6B shows the distal portion of the expandable sheath 501 in a compressed state, wherein its distal diameter D2 is smaller than D1. It should be noted that compressing the external covering layer 561, from an un-compressed state to a compressed state of the expandable sheath 501, results in formation of folds 563 (FIGS. 44 and 46) along the external covering layer 561 as well as polymeric layers 517 and 513, when reaching the compressed state, due to the diameter reduction thereof. It is desirable to promote moderate attachment between the folds 563. The term "moderate attachment", as used herein, refers to an attachment force sufficient in magnitude to form a structural cover maintaining the expandable sheath 501 in a compressed state prior to advancement of a DS component through its lumen, yet low enough so that advancement of the DS component there-through is sufficient to break or disconnect the attachments 565 between the folds 563 (FIG. 44), thereby enabling expansion of the expandable sheath 501.

The external covering layer 561 is chosen such that its melting temperature TM1 is lower than the melting temperature TM2 of the polymeric layers of the expandable sheath 100, in order to promote folds 563 formation with moderate attachment in the external covering layer 561, while avoiding melting and attaching similar folds in the polymeric layers 513 and 517 of the expandable sheath 501.

According to some aspects, the external covering layer 561 is low density polyethylene. Other suitable materials as known in the arts, such as polypropylene, thermoplastic polyurethane, and the like, may be utilized to form the external covering layer 561.

FIGS. 45 and 46 show perspective views of a sheath aspect that is similar to or the same as FIGS. 43 and 44. The external covering layer 561 and expandable sheath 501were heated to a first temperature TM2 along a circumferential interface there between at the proximal end of the external covering layer 561, to form a circumferential proximal attachment region 569.

According to some aspects, the external covering layer 561 is attached different attachment regions, such as along a longitudinally oriented attachment line, to the external surface of the expandable sheath 501 (e.g. the outer polymeric layer). According to some aspects, the external covering layer 561 is attached to the external surface of the expandable sheath 501 by a plurality of circumferentially spaced attachment regions 569, 571, wherein the circumferential distance between adjacent attachment regions is chosen to allow formation of folds 573 there between. Attachment regions 569, 571 ensure that the external covering layer 561 always remains attached to the expandable sheath 501, either during the compressed or expanded states thereof.

According to some aspects, the covering with an external covering layer 561 is performed after crimping the expandable sheath 501, such that the external layer 561 covers pre-formed folds of inner 513 and/or outer 517 polymeric layers of the sheath 501.

According to some aspects, the bond between the folds 563 is based on adhesive with moderate adhesion strength.

Aspects of the sheaths described herein may comprise a variety of lubricious outer coatings, including hydrophilic or hydrophobic coatings, and/or surface blooming additives or coatings.

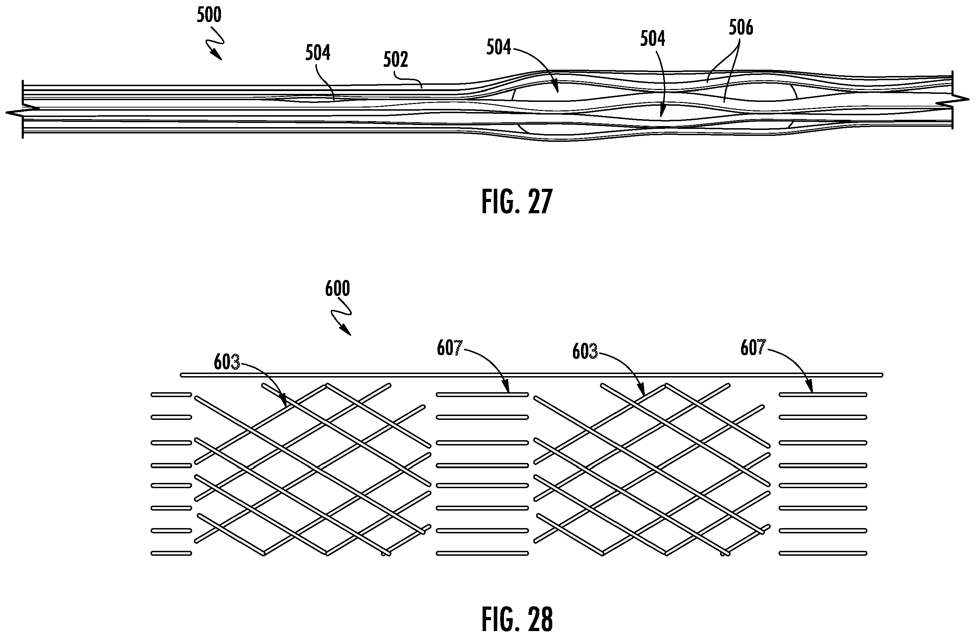

FIG. 27 illustrates another aspect of a sheath 500 comprising a tubular inner layer 502. The inner layer 502 may be formed from an elastic thermoplastic material such as nylon, and can comprise a plurality of cuts or scorelines 504 along its length such that the tubular inner layer 502 is divided into a plurality of long, thin portions or ribs 506. When the delivery apparatus 10 is advanced through the tubular inner layer 502, the scorelines 504 can resiliently expand or open, causing the ribs 506 to splay apart, and allowing the diameter of the inner layer 502 to increase to accommodate the delivery apparatus.

In other aspects, the scorelines 504 can be configured as openings or cutouts having various geometrical shapes, such as rhombuses, hexagons, etc., or combinations thereof. In the case of hexagonal openings, the openings can be irregular hexagons with relatively long axial dimensions to reduce foreshortening of the sheath when expanded.

The sheath 500 can further comprise an outer layer (not shown), which can comprise a relatively low durometer, elastic thermoplastic material (e.g., Pebax, polyurethane, etc.), and which can be bonded (e.g., by adhesive or welding, such as by heat or ultrasonic welding, etc.) to the inner nylon layer. Attaching the outer layer to the inner layer 502 can reduce axial movement of the outer layer relative to the inner layer during radial expansion and collapse of the sheath. The outer layer may also form the distal tip of the sheath.

FIG. 28 illustrates another aspect of a braided layer 600 that can be used in combination with any of the sheath aspects described herein. The braided layer 600 can comprise a plurality of braided portions 603, in which filaments of the braided layer are braided together, and unbraided portions 607, in which the filaments are not braided, and extend axially without being intertwined. In certain aspects, the braided portions 603 and unbraided portions 607 can alternate along the length of the braided layer 600, or may be incorporated in any other suitable pattern. The proxportion of the length of the braided layer 600 given to braided portions 603 and unbraided portions 607 can allow the selection and control of the expansion and foreshortening properties of the braided layer.

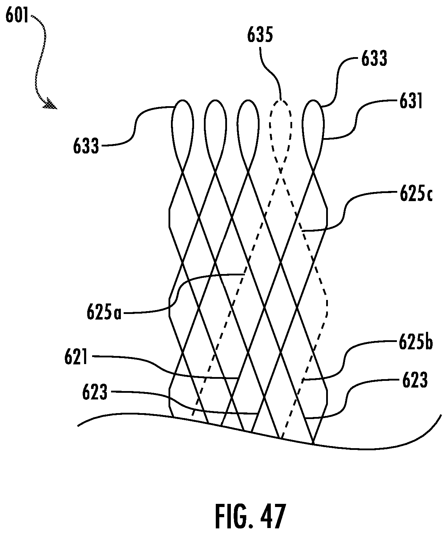

FIG. 47 depicts an aspect of a braided layer having at least one radiopaque strut or filament. The expandable sheath 601 and its expandable braided layer 621 is shown without the polymeric layers, as would be visualized in the x-ray fluoroscopy, for purposes of illustration. As shown in FIG. 47, the expandable braided layer 621 comprises a plurality of crossing struts 623, that can further form distal crowns 633, for example in the form of distal loops or eyelets at the distal portion of the expandable sheath 601.

The expandable sheath 601 is configured for advancement in a pre-compressed state up to a target area, for example along the abdominal aorta or the aortic bifurcation, at which point the clinician should cease further advancement thereof and introduce the DS through its lumen, to facilitate expansion thereof. For that end, the clinician should receive a real-time indication of the expandable sheath's position during advancement thereof. According to an aspect of the disclosure, there is provided at least one radio-opaque marker at or along at least one region of the expandable braided layer 621, configured to enable visualization of the expandable sheath's position under radio fluoroscopy.

According to one aspect, at least one of the distal crowns 633 comprises a radio-opaque marker. According to some aspects, the distal crowns 633 comprise at least one gold-plated crown 635 (FIG. 47), configured to serve as a radio-opaque marker. It will be clear that gold-plating is merely an example, and that the crowns 635 can comprise other radio-opaque material known in the art, such as tantalum, platinum, iridium and the like.

Since the expandable sheath 601 comprises an expandable braided layer 621 having a plurality of crossing struts 623 disposed along its length, this structure can be advantageously utilized for more convenient incorporation of radio-opaque elements.

According to some aspects, the struts 623 further comprise at least one radio-opaque strut 625, having a radio-opaque core. For example, a drawn filled tubing (DFT) wire comprising a gold core (as may be provided by, for example, Fort Wayne Metals Research Products Corp.) may serve as a radio-opaque strut 625. FIG. 47 shows an exemplary expandable braided layer 621 comprising a plurality of less-opaque struts or filaments 623 and radio-opaque struts or filaments 625a, 625b and 625c. In some instances, the struts 625a and 625c can be made of a single wire, wherein the wire extends along the path of strut 625a, loops at the distal crown 635 and extends along the path of strut 625c therefrom. Thus, a single wire, such as a DFT wire, can be utilized to form radio-opaque struts 625a and 625c and radio-opaque distal crown 635.

Since radio-opaque wires, such as a DFT wire, can be costly, the expandable braided layer 621 can comprise a plurality of non radio-opaque or less radio-opaque struts 623, for example made of a superelastic alloy such as Nitinol and polymer wire such as PET respectively, intertwined with at least one radio-opaque strut 625 (FIG. 47).

According to some aspects, radio-opaque wires are embedded within the polymer braid, such as the outer polymeric layer 617 or the inner polymeric layer 615, which are made of less-opaque materials.

Advantageously, the expandable braid embedded within the expandable sheath is utilized according to the disclosure, for incorporating radio-opaque markers along specific portions thereof to improve visualization of the sheath's position in real- time under radio fluoroscopy.

According to yet another aspect of the disclosure, radiopaque tubes can be threaded on the distal crowns or loops 633, or radiopaque rivets can be swaged on the distal crowns or loops 633 to improve their visibility under fluoroscopy.

FIG. 36 shows a longitudinal cross section of another aspect of expandable sheath 11 (positioned on mandrel 91 during the fabrication process, under compression by heat shrink tube 51). The sheath 11 comprises a braided layer 21, but lacks the elastic layer described in the previous aspects. The heat applied during the shrinking procedure may promote at least partial melting of the inner and outer polymeric layers 31, 41. Since the filaments of the braid define open cells therebetween, uneven outer surfaces may be formed when the inner and outer polymeric layers 31, 41 melt into the cell openings and over the filaments of the braided layer 21.

In order to mitigate uneven surface formations, cushioning polymeric layers 61a, 61b are added between the inner and outer layers 31, 41 of the sheath 11, configured to evenly spread the forces acting in the radial direction during sheath compression. A first cushioning layer 61a is placed between the inner polymeric layer 31 and the braided layer 21, and a second cushioning layer 61b is placed between the outer polymeric layer 41 and the braided layer 21. In some aspects, the cushioning polymeric layers 61a and 61b are sacrificial and are removed in a later processing step.

The cushioning layers 61a, 61b can comprise a porous material having plurality of micropores of nanopores 63 (FIGS. 37-38) in a porous interior region. One such material includes, but is not limited to, expanded polytetrafluoroethylene (ePTFE). A porous cushioning layer can advantageously be formed with a minimal thickness *h1* required to sufficiently spread the compression forces to prevent uneven surface formation along the inner and outer polymeric layers 31, 41. Thickness *h1* is measured in the radial direction (from an inner surface to an outer surface) of the cushioning layer, and can be from about 80 microns to about 1000 microns (including, for example, about 80 microns, about 90 microns, about 100 microns, about 110 microns, about 120 microns, about 130 microns, about 140 microns, about 150 microns, about 160 microns, about 170 microns, about 180 microns, about 200 microns, about 250 microns, about 300 microns, about 350 microns, about 400 microns, about 450 microns, about 500 microns, about 550 microns, about 600 microns, about 650 microns, about 700 microns, about 750 microns, about 800 microns, about 850 microns, about 900 microns, about 950 microns, and about 1000 microns). In some aspects, the range of thickness *h1* is from about 110 to 150 microns.

However, when cushioning layers comprise a plurality of micropores of nanopores 63 (FIGS. 37-38), the inner and outer polymeric layers 31, 41 may melt into the pores of the cushioning layers 61a, 61b upon heating during the fabrication process. In order to prevent the inner 31 and outer 41 polymeric layers from melting into the nanopores 63 of the cushioning layer 61, a first sealing layer 71a can be placed between the inner polymeric layer 31 and the first cushioning layer 61a, and a second sealing layer 71b can be placed between the outer polymeric layer 41 and the second cushioning layer 61b. (as shown in FIG. 36). The sealing layers 71a, 71b can have a higher melting point than the polymeric layers 31 and 41, and can be formed of a non-porous material (such as, but not limited to, polytetrafluoroethylene) in order to prevent fluid flow therethrough. The thickness h2 of each sealing layer 71 (FIG. 37), measured in a radial direction from the inner to the outer surface of the sealing layer, can be much thinner than that of the cushioning layer 61, for example, from about 15 to about 35 microns (including about 15 microns, about 20 microns, about 25 microns, about 30 microns, and about 35 microns). In some aspects, the sealing layers 71a and 71b are sacrificial and are removed in a later processing step.

While advantageous for the reasons described above, the addition of the cushioning and sealing can increase the complexity and time required to assemble the sheath 11. Advantageously, providing a single sealed cushioning member, configured to provide both cushioning and sealing functionalities (instead of providing two separate cushioning and sealing layers, each configured to provide one functionality) reduces sheath assembly time and significantly simplifies the process. According to an aspect of the disclosure, there is provided a single sealed cushioning member, configured for placement between the inner and outer polymeric layers of the sheath and the central braided layer. The single sealed cushioning member includes cushioning layer and a sealed surface configured to prevent leakage/melting into the pores in the radial direction.

FIG. 37 shows an aspect of a single sealed cushioning member 81', comprising a cushioning layer 61 having a width thickness *h1* as elaborated herein above, fixedly attached to a corresponding sealing layer 71 having a thinner thickness h2 to form the sealed surface. The sealing layer 71 and the cushioning layer 61 are pre-assembled or pre-attached to each other to form together a single sealed cushioning member 81', for example by gluing, welding and the like.

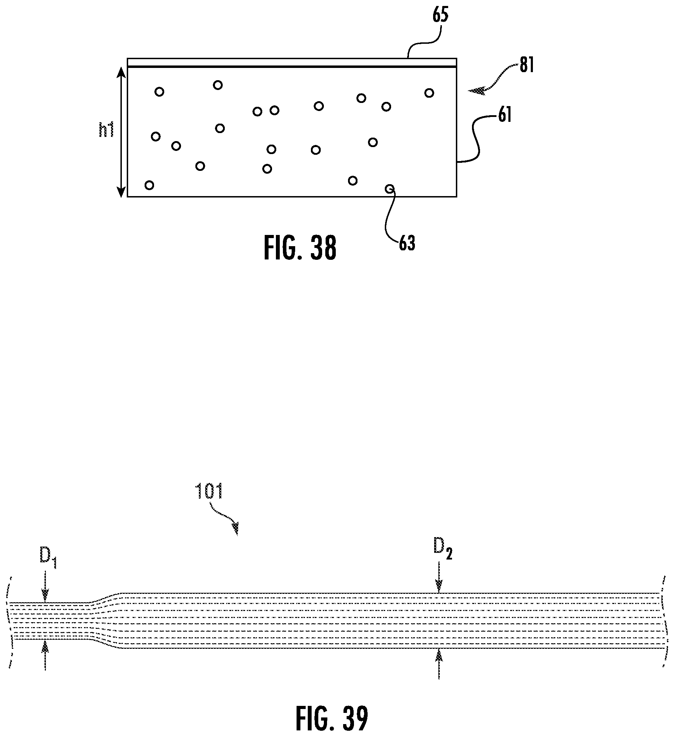

FIG. 38 shows one aspect of a single sealed cushioning member 81, comprising a cushioning layer 61 having a width thickness *h1,* wherein the cushioning layer 61 is provided with at least one sealed surface 65, configured to face an inner 31 or an outer 41 polymeric layer when assembled in the sheath 11. According to some aspects, the sealed surface 65 can be formed by a surface treatment configured to fluidly seal a surface of the cushioning layer 61. As such, the sealed surface 65 can be the same material as the cushioning layer 61.

According to another aspect of the disclosure, and as mentioned above with respect to FIG. 36, a minimum of three layers may be sufficient to retain the sheath's expandability provided with the preferable resistance to axial elongation. This is accomplished by eliminating the need to incorporate an additional elastic layer in the sheath, thereby advantageously reducing production costs and simplifying manufacturing procedures. The sheath does not necessarily return to an initial diameter, but may rather remain in an expanded diameter upon passage of the valve, in the absence of the elastic layer.

FIGS. 39-40 show an expandable sheath 101 similar to the expandable sheath 100 shown in FIG. 3, but without an elastic layer 106. The inner and outer layers 103 and 109 may be structured and configured to resist axial elongation of the sheath 101 during expansion. However, in the proposed configuration, the absence of an elastic layer results in the sheath 101 remaining in an expanded diameter along the sheath's portion proximal to the valve, without necessarily collapsing back to the initial diameter D₁ after the valve passes in in the longitudinal direction. FIG. 39 is a schematic representation of the sheath 101 remaining in an expanded diameter D₂ along the portion proximal to the valve's passage.

Thus, there is provided an expandable sheath for deploying a medical device, comprising a first polymeric layer, a braided layer radially outward of the first polymeric layer, and a second polymeric layer radially outward of the braided layer. The braided layer includes a plurality of filaments braided together. The second polymeric layer is coupled to the first polymeric layer such that the braided layer is encapsulated between the first and second polymeric layers. When a medical device is passed through the sheath, the diameter of the sheath expands from a first diameter to a second diameter around the medical device, while the first and second polymeric layers resist axial elongation of the sheath such that the length of the sheath remains substantially constant. However, according to some aspects, the first and second polymeric layers are not necessarily configured to resist axial elongation.

According to another aspect of the disclosure, the expandable sheath does include an elastic layer. But, unlike elastic layer 106 shown in FIG. 3, the elastic layer is not configured to apply a substantial radial force. It can still serve to provide column strength to the sheath. By limiting tangential (diametrical) expansion of the braid, the elastic layer enhances the strength of the braid and the sheath in the axial direction (column strength). As such, use of elastic materials with higher tensile strengths (resistance to stretch) will result in a sheath with greater column strength. Likewise, elastic materials that are under greater tension in the free state will also result in a sheath with greater column strength during pushing, as they will be more resistant to stretch. The pitch of any helically wound elastic layers is another variable that contributes to the column strength of the sheath. The additional column strength ensures that the sheath does not spontaneously expand due to frictional forces applied thereto during forward movement in a distal direction, and does not buckle when the delivery system is pulled out of the sheath.

In another optional aspect, the elastic layer can be applied by dip coating in an elastic material (such as, but not limited to) silicone or TPU. The dip coating can be applied to the polymeric outer layer, or to the braided layer.

Thus, there is provided an expandable sheath for deploying a medical device, comprising a first polymeric layer, a braided layer radially outward of the first polymeric layer, an elastic layer radially outward of the braided layer, and a second polymeric layer radially outward of the braided layer. The braided layers comprise a plurality of filaments braided together. The elastic layer is configured to provide the expandable sheath with sufficient column strength to resist buckling of spontaneous expansion due to friction forces applied thereto by a surrounding anatomical structure during the sheath's movement in an axial direction. The second polymeric layer is coupled to the first polymeric layer such that the braided layer is encapsulated between the first and second polymeric layers. When a medical device is passed through the sheath, the diameter of the sheath expands from a first diameter to a second diameter around the medical device, optionally while the first and second polymeric layers resist axial elongation of the sheath such that the length of the sheath remains substantially constant.

According to an aspect of the disclosure, there is provided a three-layered expandable sheath, comprising an inner polymeric layer, an outer polymeric layer coupled to the inner polymeric layer and a braided layer encapsulated between the inner and outer polymeric layers, wherein the braided layer comprises an elastic coating.

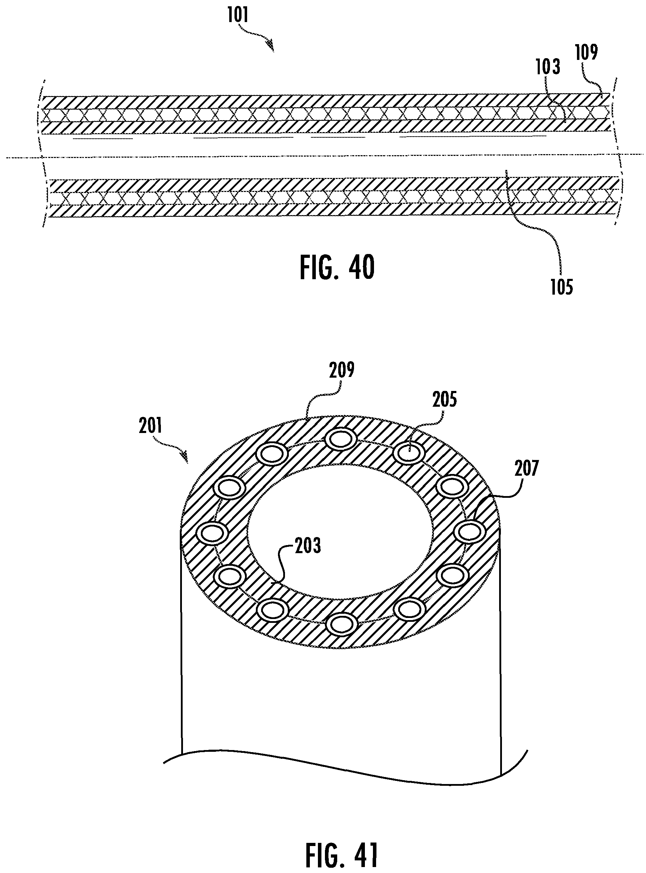

FIG. 41 shows a transverse cross section of expandable sheath 201. The expandable sheath 201 includes inner and outer polymeric layers 203 and 209 and a braided layer 205. Instead of the elastic layer described with reference to FIG. 3, above, the braided layer 205 is provided with an elastic coating 207. The elastic coating 207 can be applied directly to the filaments of the braided layer 205, as shown in FIG. 41. The elastic coating can be made of synthetic elastomers, exhibiting properties similar to those described in conjunction with the elastic layer 106.

In some aspects, the second, outer polymeric layer 209 is coupled to the first, inner polymeric layer 203 such that the braided layer 205 and the elastic coating 207 are encapsulated between the first and second polymeric layers. Moreover, the elastic coating applied directly to the braided filaments is configured to serve the same function as that of the elastic layer 106 (that is, to apply radial force on the braided layer and the first polymeric layer).

While the aspect of FIG. 41 shows the elastic coating 207 covering the entire circumference of every filament of the braided layer 205, it will be understood that only a portion of the filaments, for example, a portion constituting essentially an outer surface of the braided layer, may be coated by the elastic coating 207.

Alternatively or additionally, an elastic coating can be applied to other layers of the sheath.

In some aspects, a braided layer 105 such as the one shown in FIG. 39 and FIG. 40 can have self-contractible frame made of a shape-memory material, such as, but not limited to, Nitinol. The self-contracting frame can be pre-set to have a free-state diameter equal to the sheath's initial compressed diameter D1, for example, prior to being placed on a mandrel around the first polymeric layer. The self-contracting frame may expand to a larger diameter D2 while an inner device, such as a prosthetic valve, passes through the sheath's lumen, and self-contract back to the initial diameter D1 upon passage of the valve. In some aspects, the filaments of the braid are the self-contracting frame, and are made of a shape-memory material.

According to another aspect, an expandable sheath can include a braided expandable layer, attached to at least one expandable sealing layer. In some aspects, the braided layer and the sealing layer are the only two layers of the expandable sheath. The braided layer is passively or actively expandable relative to a first diameter, and the at least one expandable sealing layer is passively or actively expandable relative to a first diameter. An expandable sealing layer can be useful with any of the aspects described above, and may be particularly advantageous for braids having self-contracting frames or filaments.

The braided layer can be coupled or bonded to the expandable sealing layer along its entire length, advantageously decreasing the risk of the polymeric layer being peeled off the braided layer due to frictional forces that may be applied thereon either during entry or exit through the surgical incision. The at least one sealing layer can comprise a lubricious, low-friction material, so as to facilitate passage of the sheath within the blood vessels, and or to facilitate passage of the delivery apparatus carrying a valve through the sheath.

A sealing layer is defined as a layer which is not permeable to the blood flow. The sealing layer can comprise a polymeric layer, a membrane, a coating and/or a fabric, such as a polymeric fabric. According to some aspects, the sealing layer comprises a lubricious, low-friction material. According to some aspects, the sealing layer is radially outward to the braided layer, so as to facilitate passage of the sheath within the blood vessels. According to some aspects, the sealing layer is radially inward to the braided layer, so as to facilitate passage of the medical device through the sheath.

According to some aspects, the at least one sealing layer is passively expandable and/or contractible. In some aspects, the sealing layer is thicker at certain longitudinal positions of the sheath than at others, which can hold a self-contracting braided layer open at a wider diameter than at other longitudinal positions where the sealing layer is thinner.

Attaching the braided layer to at least one expandable sealing layer, instead of encapsulating it between two polymeric layers coupled to each other, may simplify manufacturing process and reduce costs.

According to some aspects, the braided layer can be attached to both an outer expandable sealing layer and an inner expandable sealing layer, so as to seal the braided layer from both sides, while facilitating passage of the sheath along the blood vessels, and facilitating passage of a medical device within the sheath. In such aspects, the braided layer can be attached to a first sealing layer, while the other sealing layer may be also attached to the first sealing layer. For example, the braided layer and the inner sealing layer can be each attached to the outer sealing layer, or the braided layer and the outer sealing layer can be each attached to the inner sealing layer.

According to some aspects, the braided layer is further coated by a sealing coating. This may be advantageous in configurations of a braided layer being attached only to a single expandable layer, wherein the coating ensures that the braided layer remains sealed from the blood flow or other surrounding tissues, even along regions which are not covered by the expandable layer. For example, if a braided layer is attached to a sealing layer on one side, the other side of the braided layer may receive a sealing coating. In some aspects, the sealing coating can be used instead of, or in addition to, one or both of the sealing layers.

In another aspect, an expandable sheath can include one or more radiopaque features. FIGS. 48-54 show sheath aspects that include one or more radiopaque foil layers. A radiopaque foil layer or layers can be tubular, extending circumferentially around the sheath wall. The radiopaque foil layer can be embedded within the sheath wall or adhered to the outer surface of the sheath wall as an outer layer. The radiopaque foil layer or layers can be layered between other layers of the sheath wall in any radial order. The radiopaque foil layer can extend longitudinally over any portion of the sheath (the full length, the distal portion, the proximal portion, a central portion, or any combination thereof).

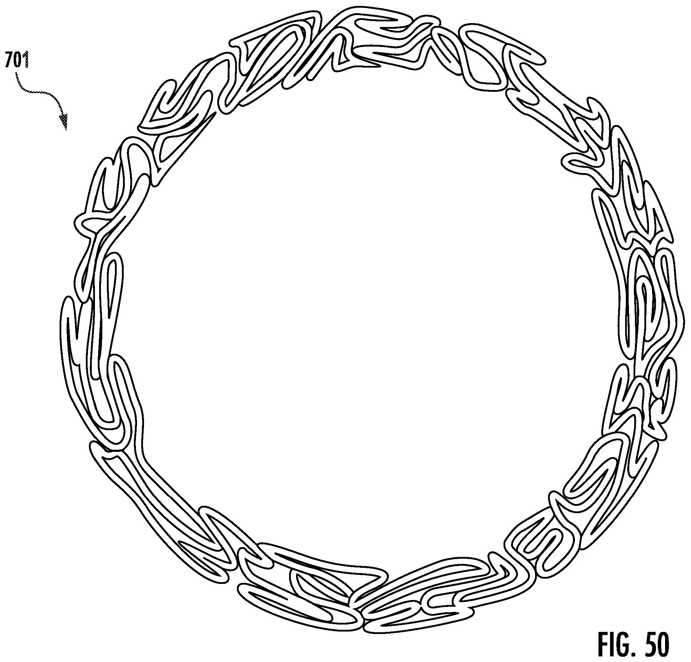

As shown in FIG. 48 and the magnified inset at FIG. 49, the collapsed sheath wall 703 can include a plurality of folds 763. FIG. 48 depicts a cross section adjacent the distal tip of the sheath, and as such does not show braided layer 704 which stops short of the distal tip (as in, for example, FIG. 46). The folds 763 can extend longitudinally along the entire length of sheath 701, along just a distal portion of sheath 701, along just a central portion, along just a proximal portion, or along any combination thereof. When the sheath 701 is in a collapsed state, the longitudinally extending folds 763 form a plurality of longitudinally extending ridges circumferentially spaced apart from each other by longitudinally extending valleys. The folds can be arranged in an orderly fashion, as shown in FIG. 49, but are not necessarily so. In some aspects, manufacturing processes lead to more randomly arranged longitudinally extending folds, valleys, and ridges, such as is shown in FIG. 50. FIG. 50 also depicts a cross section adjacent the distal tip of the sheath, and as such does not show braided layer 704 which stops short of the distal tip (as in, for example, FIG. 46).

Advantageously, the radiopaque foil layer 705 of the sheath wall 703 is sufficiently thin to allow the sheath wall to be folded longitudinally. For example, the foil layer 705 might have a thickness ranging anywhere from about 5 microns to about 50 microns thick, or anywhere from about 10 microns to about 30 microns thick (including, for example, about 5 microns, about 10 microns, about 15 microns, about 20 microns, about 25 microns, about 30 microns, about 35 microns, about 40 microns, about 45 microns and about 50 microns thick).

Sheath wall 703 is more readily detected by imaging modalities in its collapsed, folded state due to a higher radiopacity than the unfolded, expanded state. The increased radiopacity of the folded state is attributed to the increased density of the radiopaque foil layer 705. For example, as shown in FIG. 49, a single layer of the sheath wall 703 has a thickness ***t***. Folded sheath wall thickness ***T*** can be defined as the distance between the innermost surface of the sheath (closest to the lumen) and the outermost surface of the sheath at a given circumferential and longitudinal point. The full thickness ***T*** of the folded sheath wall can vary due to the valleys and ridges formed by the folds, but is higher than ***t***, the thickness of a single sheath wall layer. The folded sheath wall 703 is sufficiently dense that it is readily detected using medical imaging modalities, such as, for example X-ray and fluoroscopy. As a medical device passes through the inner lumen of the sheath, it applies an outward radial force on the sheath wall 703. This causes the plurality of longitudinally extending folds 763 to partially or fully unfold (that is, a single longitudinally extending fold may partially or fully unfold, several of the longitudinally extending folds may partially or fully unfold, or all of the longitudinally extending fold may partially or fully unfold). The sheath wall 703 thickness therefore decreases from ***T*** to ***t*** during expansion from a fully collapsed, folded state to a fully expanded, unfolded state. The decrease in thickness translates to a decrease in density and can be detected by an imaging modality as a decrease in radiopacity.

The radiopaque foil layer 705 can be positioned between other layers of the sheath wall 703. For example, FIG. 49 shows a radiopaque foil layer 705 encapsulated by a first, inner polymeric layer 707 and a second, outer polymeric layer 709. The radiopaque foil layer 705 is positioned radially outward of the tubular first polymeric layer 707 that defines the inner lumen of the expandable sheath 701. An additional second polymeric layer 709 is positioned radially outward of the radiopaque foil layer 705. In some aspects, additional layers can be positioned between the foil layer 705 and the inner polymeric layer 707, and/or between the foil layer 705 and the outer polymeric layer 709. Exemplary materials for the inner and outer polymeric layers 707, 709 can include ultra-high-molecular-weight polyethylene (UHMWPE) (e.g., Dyneema^{®}), high-density polyethylene (HDPE), or polyether ether ketone (PEEK). With regard to the inner layer 707, low coefficient of friction materials can facilitate passage of the medical device through the inner lumen. Other suitable materials for the inner and outer layers can include polytetrafluoroethylene (PTFE), expanded polytetrafluoroethylene (ePTFE), ethylene tetrafluoroethylene (ETFE), nylon, polyethylene, polyether block amide (*e.g.*, Pebax), and/or combinations of any of the above.

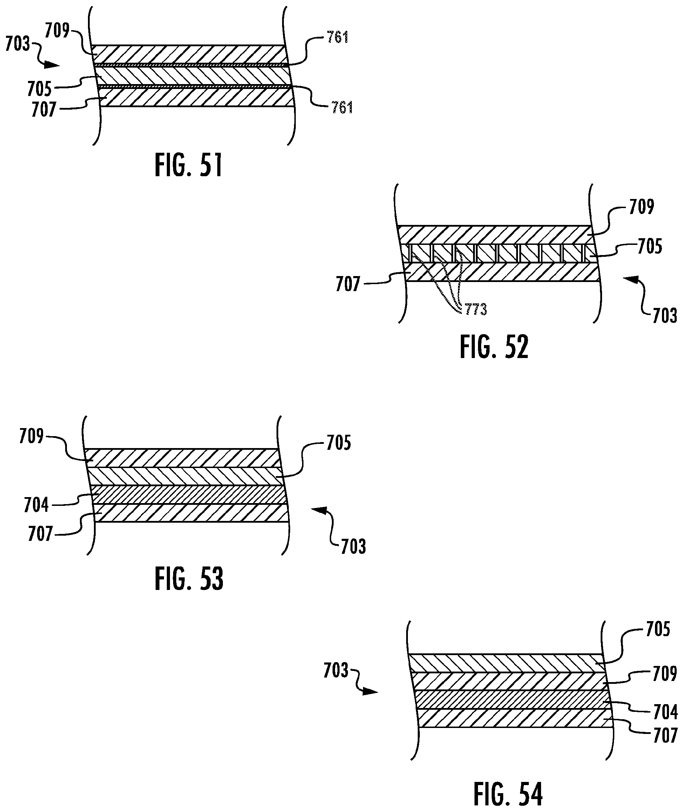

To improve lamination of the radiopaque foil layer 705 to the braided layer 704 and increase mechanical strength, in some aspects the radiopaque foil layer 705 is laminated to an adjacent layer, bonded to an adjacent layer using an adhesive 761 as shown in FIG. 51, and/or adhered to an adjacent layer using a tie layer. Laminates and adhesives can include, for example, low-density polyethylene (LDPE) and hot glue. A tie layer can be formed of ethylene vinyl acetate (EVA), for example.

In some aspects, the radiopaque foil layer 705 includes openings 773 extending in in a radial direction through the foil layer 705. The openings 773 facilitate bonding of adjacent inner and outer polymeric layers 707, 709 through the foil layer 705 as shown in FIG. 52. For example, a first polymeric layer 707 may be positioned radially inward of the foil layer 705 and a second polymeric layer 709 positioned radially outward of the foil layer. The first and second polymeric layers can be coupled together, thermally, for example, through the openings 773 of the foil layer 705. The openings 773 can be shaped and patterned to maximize bonding while ensuring the foil layer 705 is still detectable by imaging modalities when folded. For example, the openings 773 can be patterned as an array that extends circumferentially around the foil layer 705. In some aspects, the openings 773 can be formed using laser cutting. The radiopaque material can be disposed along the whole length of the sheath.

In some aspects, the radiopaque foil layer 705 may be incorporated into a sheath wall 703 that also includes a braided layer 704 such as those described above. The radiopaque foil layer 705 can be positioned radially outward of the braided layer 704, or radially inward of the braided layer 704. In the aspect shown in FIG. 53, the braided layer 704 is positioned radially outward from the first polymeric layer 707, and the foil layer 705 is positioned radially outward from the braided layer 704. The second polymeric layer 709 is positioned radially outward from the foil layer 705. Alternatively, the foil layer 705 can be positioned radially outward from the first polymeric layer 707, the braided layer 704 can be positioned radially outward from the foil layer, and the second polymeric layer 709 is positioned radially outward from the braided layer 704. In some aspects, adjacent polymeric layers can be coupled to each other through the filaments of the braided layer 704 and/or through openings 773 of the foil layer 705.

Other variations in layering are possible. For example, as shown in FIG. 54, the radiopaque foil layer 705 can be positioned on the outer surface of the sheath wall 703, regardless of whether a braided layer 704 is also included. A laminate or adhesive can be used to adhere foil layer 705 to the outer surface of sheath wall 703. In some aspects, both a foil layer 705 and a braided layer 704 are included, but along different longitudinal sections of the sheath wall 703. In some aspects, additional polymeric layers of varying materials can be included to vary sheath wall properties such as, for example, push force, kink resistance, resistance to ballooning, and/or bending to any angle or diameter.

The foil layer 705 can be made from, or can include, a radiopaque metal, such as (but not limited to), platinum, gold, silver, tin, copper, iridium, palladium, tantalum, mixtures or alloys thereof. The radiopaque metal material can form the foil, or can be mixed or otherwise incorporated into (or onto the surface of) another material as a powder.

Aspects including a radiopaque foil layer can also include an outer cover formed of a heat shrink material, as described in reference to FIG. 23. For example, the outer cover can extend over at least a longitudinal portion of the first polymeric layer and the foil layer. The outer cover can include one or more longitudinally extending slits, weakened portions, or scorelines. Furthermore, aspects including a radiopaque foil layer can also include an elastic outer layer that applies an inward radial force on the sheath wall, biasing the sheath toward the collapsed state, as described above.

Other types of radiopaque features can also be incorporated into the expandable sheaths disclosed herein. Radiopaque features can be incorporated radially outward of a first polymeric layer, either over, underneath, or coupled to a braided layer that is positioned over the first polymeric layer. A second polymeric layer can be positioned radially outward of the braided layer and the radiopaque feature. The second polymeric layer can be coupled to the first polymeric layer such that the braided layer and the radiopaque feature are encapsulated between the first and second polymeric layers.

In some aspects, the radiopaque feature can be a radiopaque polymer layer. Polymers can be made radiopaque through enhancing (blending, doping, or surface treating) with radiopaque agents such as, but not limited to, barium sulfate, bismuth, tantalum, iodine, or tungsten. The radiopaque agents are frequently provided in powder form before mixing with or surface treating the polymer, but that is not always the case. For example, a polypropylene film or sheet can be doped with or surface-treated on one or both sides with a radiopaque powder such as barium sulfate. As another example, an ePTFE sheet can be doped with or surface-treated on one or both surfaces with a radiopaque metal. Use of a radiopaque polymer layer as the radiopaque feature can be advantageous because it can be made very thin, contributing little to the overall profile of the sheath. A radiopaque polymer layer can also be provided relatively inexpensively.

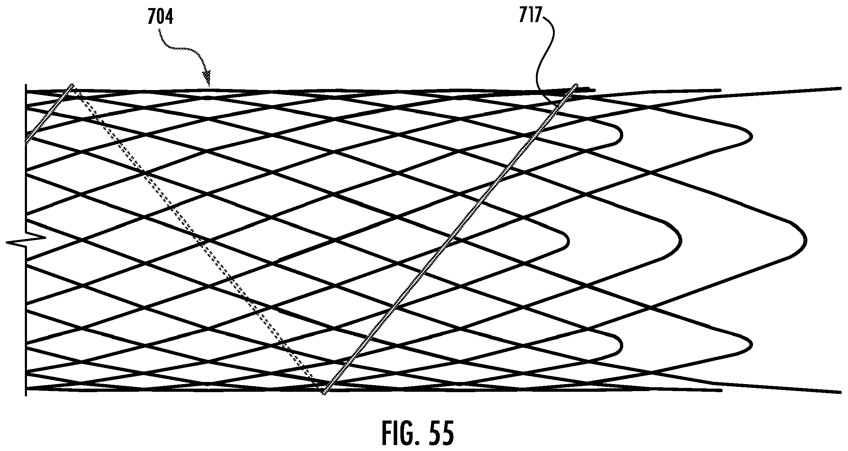

In some aspects, the radiopaque feature is one or more longitudinally extending, radiopaque cords, wires, or sutures. For example, see FIG. 55. A single longitudinally extending cord 717 winds helically around the outer surfaces of braided layer 704. In some aspects, the cord 717 could extend underneath the braided layer 704 (over the first polymeric layer), or over the outside of a second polymeric layer positioned over the braided layer 704. In some aspects, the cord or cords 717 might be woven through the filaments of the braided layer 704. While FIG. 55 shows a radiopaque cord 717 wrapped helically around the braided layer 704, in other aspects the cord 717 can extend substantially parallel to a longitudinal axis of the sheath. In other aspects, the pitch of the helically wound cord 717 can be lower or higher than is shown in FIG. 55. In yet further aspects, the pitch of the helically wound cord 717 matches the pitch of the braid. The cord 717 can extend the entire length of the sheath, or it can extend just a portion of the length of the sheath. The radiopaque cord 717 can be formed of a radiopaque metal, a radiopaque polymer, or a textile doped with radiopaque material.

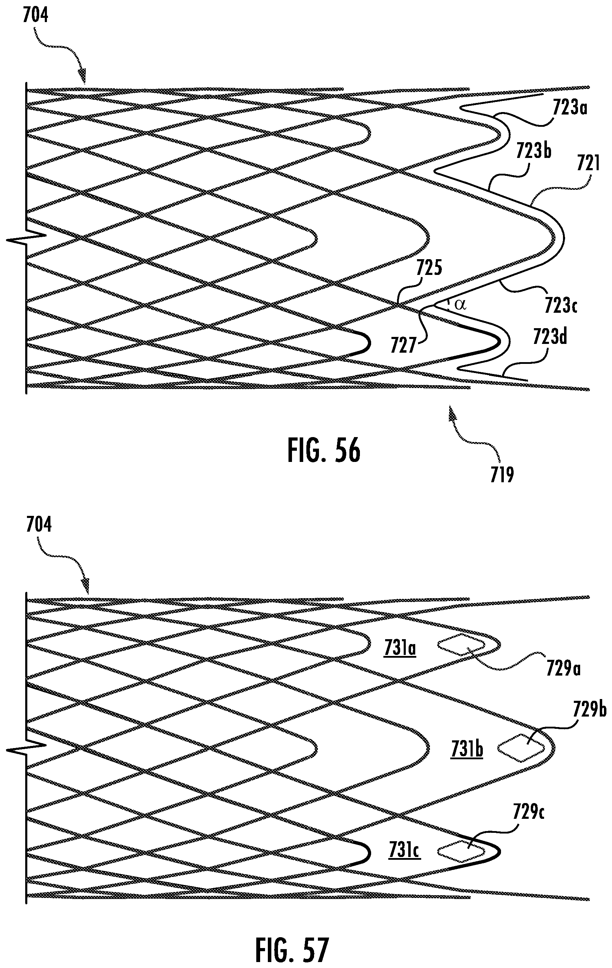

In some aspects, the radiopaque feature is encapsulated between the first and second polymeric layers without directly contacting the braided layer. This positioning can be advantageous because the placement of the radiopaque feature does not interfere with the expansion of the braided layer 704. For example, FIG. 56 shows a braided layer 704 having a radiopaque feature positioned distal to the distal end 719 of the braided layer 704. The radiopaque feature is a zig-zagged ring 721 formed of a series of circumferentially extending chevrons 723a, 723b, 723c, and 723d. While the chevrons 723 are interconnected in a zig-zagged ring 721 in FIG. 56, the chevrons 723 could alternatively be individually encapsulated between the first and second polymeric layers, adjacent to the distal end 719 of the braided layer 704, without being attached to each other. The inner angle α of a chevron 723 can increase during the passage of medical device, facilitating expansion of the sheath. The number of chevrons incorporated can be matched to, or can be less than, the number of filament crossings 725 at the distal end 719 of the braided layer 704. Generally, the apex 727 of a chevron 723 will nest near a filament crossing 725 such that the apex 727 points into a trough defined by the distal end 719 of the braided layer 704. The chevrons 723 can be formed of any radiopaque material. They can, for example, be laser cut from a sheet of radiopaque metal, such as tantalum.

Another example of a radiopaque feature that is encapsulated between the first and second polymeric layers without directly contacting the braided layer is shown in FIG. 57. Radiopaque markers 729a, 729b, and 729c are positioned within the distal-most cells 731a, 731b, and 731c defined by the filaments of braided layer 704 to assist with detection of the distal tip of the sheath. The positioning between the filaments within the cells keeps the markers 729 from interfering with the expansion of braided layer 704. Any number of markers 729 can be included. The shape of the markers can vary, and the markers 729 can be placed at other longitudinal positions along the sheath than the ones shown in FIG. 57. The markers 729 can be formed of any radiopaque material. They can, for example, be laser cut from a sheet of radiopaque metal, such as, but not limited to, tantalum.

While the markers 729 do not directly contact the braided layer 704, they could be coupled indirectly to the braided layer 704. For example, a suture could extend through a hole in a marker 729, or to chevrons 723, to indirectly couple the radiopaque feature to a nearby filament of the braided layer 704. Or, for example, the chevrons 723 of FIG. 56 could be cut to include metal extensions that could be welded or crimped onto the distal end of the braided layer 704.

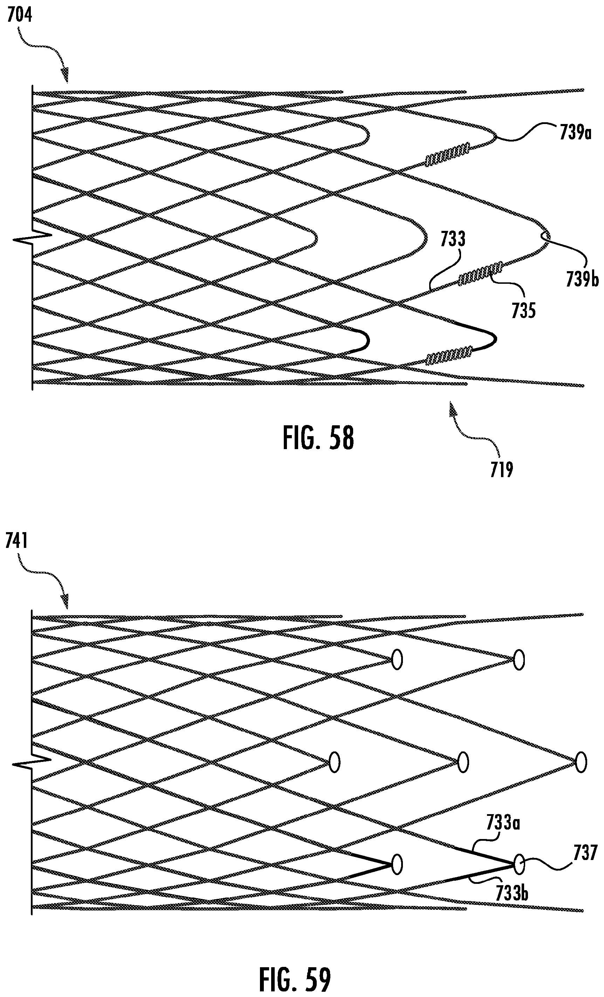

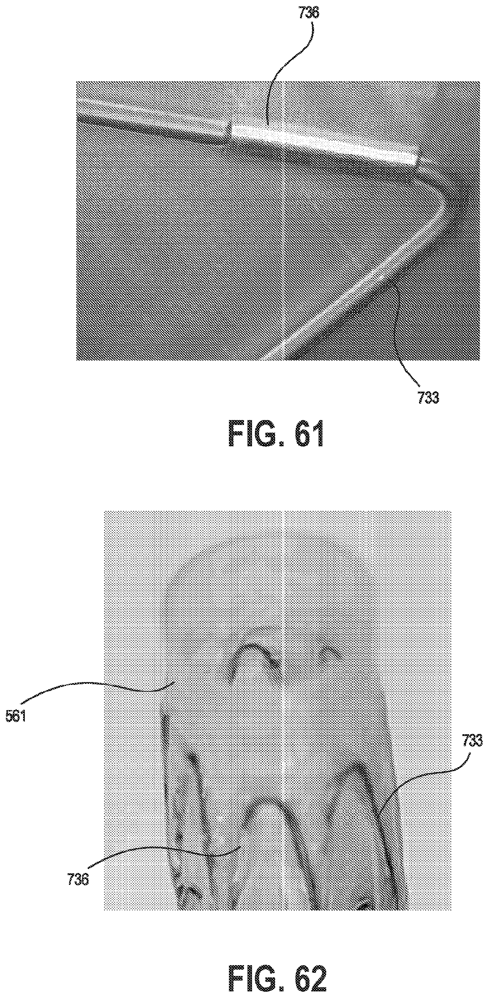

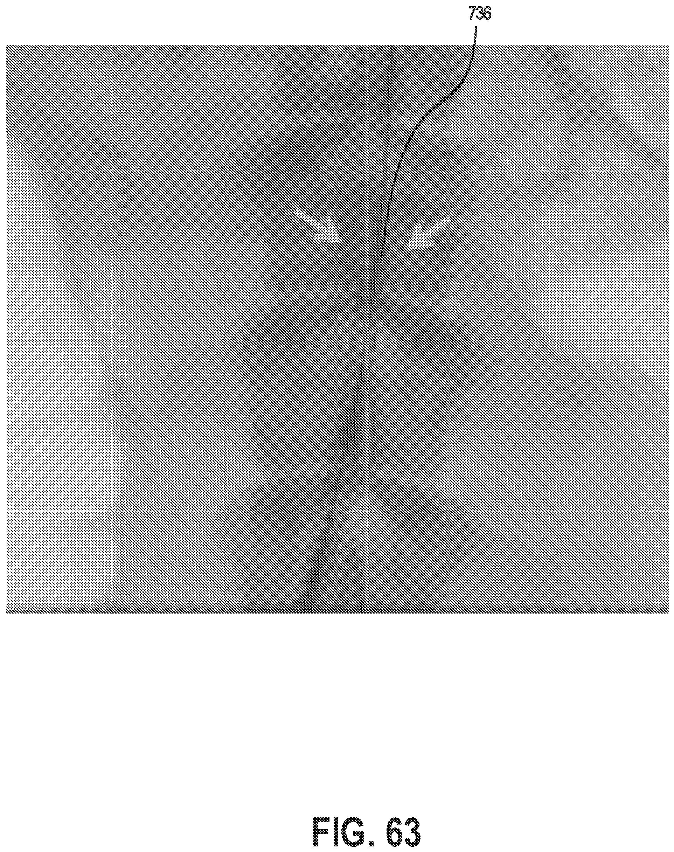

FIG. 58 shows an example wherein filament 733 of the braided layer 704 extends through a lumen defined by radiopaque coil 735. A coil 735 is shown in FIG. 58, but the radiopaque feature could also be a radiopaque tube strung onto the filament 733. For example, FIG. 61 shows a radiopaque tube 736 strung onto a filament 733. FIG. 62 shows the radiopaque tube 736 strung onto filament 733 of the braided layer 704, which is covered by an external covering layer 561. FIG. 63 shows an *in vivo* image of a sheath within the body. Imaging is facilitated by the presence of radiopaque tubes 736 near the distal end of the braided layer 704.

**In** some aspects, the radiopaque feature is secured to the braided layer 704 by some means for inducing plastic deformation, including, but not limited to, crimping, swaging, pressing, or welding. The radiopaque feature might be crimped, swaged, pressed or welded directly to the braided layer 704, directly to a distal portion of the braided layer 704, directly to the distal end 719 of the braided layer, directly to a wire that is placed at or near the distal end 719 of the braided layer 704, or directly to a wire that is threaded through the cells 731 at or near the distal end 719 of the braided layer 704 (cells 731a, 731b, 731c such as those shown in FIG. 57, for example). As an example, radiopaque coil 735 shown in FIG. 58 or radiopaque tube 736 of FIG. 61 might be crimped, swaged, pressed, or welded to the braided layer 704.

In some implementations, the radiopaque feature may be directly coupled to and/or in direct contact with the braided layer 704. For example, a radiopaque marker can include a groove. A filament of the braided layer 704 can mate with the groove of the radiopaque marker. For example, the filament can be press-fit into the groove. As another example, FIG. 59 shows an aspect wherein circumferentially adjacent filaments 733a, 733b of the braided layer 741 are joined by radiopaque beads 737. Braided layer 741 differs from the braided layer 704 shown in FIG. 58 because it has been cut from a longer braided tube. At a previous processing step (not shown), the filaments 733a, 733b were cut and had free distal ends. The radiopaque beads 737 were applied to filaments 733a, 733b to supply an atraumatic distal end to the braided layer 741. The closed loops 739 of the braided layer 704 shown in FIG. 58 are also atraumatic, but a closed loop braided layer 704 can take longer to fabricate than a free cut braided layer 741. Cutting lengths of a longer braided tube can reduce processing time by allowing for bulk fabrication of the free cut braided layer 741. Joining free distal ends of filaments 733a, 733b using radiopaque beads 737 can save time in processing while also providing a radiopaque feature. Radiopaque beads 737 can take any shape and can be formed of any radiopaque material. The beads 737 can be crimped, welded, swaged, pressed, or otherwise applied to join the distal ends of two circumferentially adjacent filaments 733a, 733b of free cut braided layer 741.

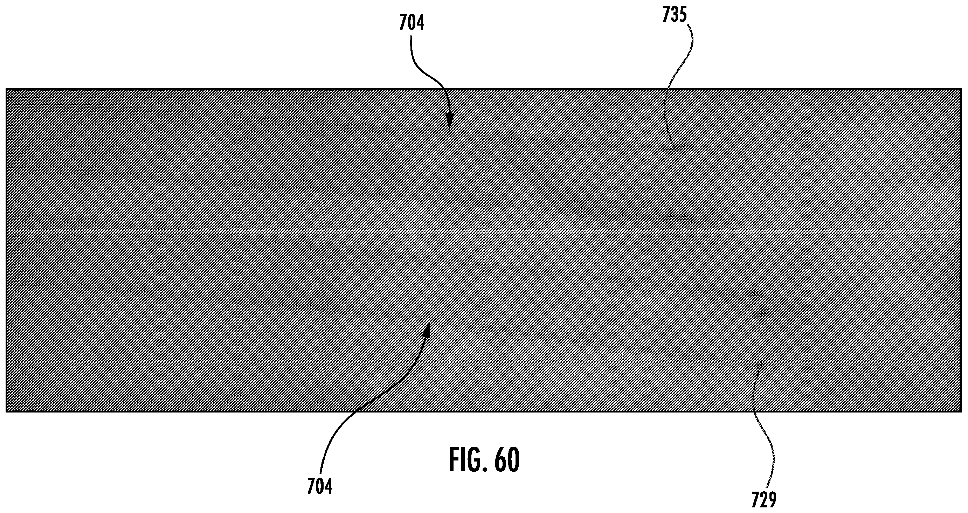

FIG. 60 shows an image of two sheaths placed on an animal for fluoroscopic image testing. The top sheath includes radiopaque coils 735 attached to a braided layer 704. The bottom sheath includes radiopaque markers 729 encapsulated between first and second polymeric layers (between cells of a braided layer 704).

It should be understood that more than one type of the radiopaque features disclosed herein can be included in the same expandable sheath.

Fabrication methods for expandable sheaths with radiopaque foil layers, such as those shown in FIGS. 48-54, will now be disclosed. As an example, a first polymeric layer 707 is positioned around a mandrel. In some aspects, a braided layer 704 is positioned radially outward of and circumferentially around the first polymeric layer 707. The braided layer 704 may be a shape memory material biased toward a collapsed configuration prior to positioning around the first polymeric layer 707. A foil layer 705 is positioned radially outward of, and circumferentially around, the first polymeric layer 707 and the braided layer 704. A second polymeric layer 709 is applied radially outwardly from the foil layer 705. A plurality of longitudinally extending folds are created in the first polymeric layer 707, the foil layer 705, and the second polymeric layer 709.

The foil layer is encapsulated between the first and second polymeric layers 707, 709. In some examples, the foil layer may be adhered to the adjacent layers, for example, using glue or adhesives. In other examples of encapsulating the foil layer, openings are cut in the foil layer 705 prior to positioning it over the first polymeric layer 707 (for example, by laser cutting). In this case, the first and second polymeric layers 707, 709 bond or melt to each other through the openings in the foil layer during a heating process (in some cases, under pressure) to encapsulate the foil layer. In some examples, the encapsulation step can involve the placement of a heat shrink wrap or tubing layer over the second polymeric layer prior to heating. Alternatively, or in addition, the encapsulation step could involve placing the mandrel with the foil layer 705 and the first and second polymeric layers 707, 709 into a vessel containing a thermally expandable material, then heating the thermally expandable material while applying a radial pressure of 100 MPa or more to the mandrel and layers via the thermally-expandable material.

The method can further comprise removing the expandable sheath 701 from the mandrel and allowing it to at least partially radially collapse to a second diameter that is less than the first diameter. This may occur, for example, due to a shape memory bias within a braided layer. In some aspects, the plurality of longitudinally extending folds are created using crimping methods which reduce the inner diameter. In some aspects, the method can further include positioning an elastic outer layer radially outward of and circumferentially around the second polymeric layer to produce a radially inward force that biases the sheath toward a smaller inner diameter.

Fabrication methods for expandable sheaths with other types of radiopaque features, such as those shown in FIGS. 55-59, will now be disclosed. The fabrication methods include positioning a braided layer 704 radially outward of, and circumferentially around, a first polymeric layer. A radiopaque feature can be positioned prior to positioning the braided layer, and/or a radiopaque feature can be positioned after positioning the braided layer. Like the braided layer, the radiopaque feature is positioned radially outward of the first polymeric layer. A second polymeric layer is applied after the braided layer and radiopaque feature(s) are positioned.

In some aspects of the fabrication methods, the radiopaque feature is a radiopaque polymer layer. Some aspects of the methods can include enhancing a polymer material with a radiopaque agent to form a radiopaque polymer prior to positioning the radiopaque polymer layer. The radiopaque polymer layer is positioned radially outward of the first polymeric layer (before or after the braided layer is positioned). The radiopaque polymer layer can be positioned or applied along the full length and circumference of the sheath, or it can be positioned or applied along only a portion of the length or circumference of the sheath. The radiopaque polymer layer might be applied directly over the first polymeric layer (and under the braided layer), or it might be applied over the braided layer. In some aspects, the radiopaque polymer layer can be applied in one or more strips which extend circumferentially, longitudinally, or helically around the sheath. In some aspects, the radiopaque polymer layer might be applied radially outward of the second polymeric layer.

In some fabrication methods, positioning the radiopaque feature comprises positioning a longitudinally extending radiopaque cord 717 such as the one shown in FIG. 55 radially outward of the first polymeric layer. This can include, for example, winding the radiopaque cord 717 helically around at least a portion of the first polymeric layer or around at least a portion of the braided layer 704. Alternatively, or in addition, a radiopaque cord could be positioned substantially parallel to the longitudinal axis of the sheath (along at least a portion of the length of the sheath). Alternatively, or in addition, the method could include weaving the radiopaque cord through filaments of the braided layer 704. Some methods can include positioning multiple radiopaque cords at various positions along the length or circumference of the sheath. The cord 717 will be encapsulated between the first and second polymeric layers. The radiopaque cord or cords 717 can also be coupled to the braided layer 704, either directly or indirectly. For example, a cord 717 can be bonded or welded to one or more filaments of the braided layer 704. Alternatively, or in addition, one or more sutures can be attached to the cord and also attached to a filament of the braided layer 704.

In some fabrication methods, the radiopaque feature is positioned such that it does not directly contact the braided layer. For example, the methods can include positioning one or more radiopaque features, such as chevrons 723, distal to the distal end 719 of a braided layer 704, as shown in FIG. 56. The radiopaque features positioned distal to the distal end 719 of the braided layer are then encapsulated between the first and second polymeric layers. Alternatively, or in addition, one or more radiopaque markers 729 can be positioned within cells 731 defined by filaments of a braided layer 704, as shown in FIG. 57. The one or more radiopaque markers 729 are then encapsulated between the first and second polymeric layers. The markers 729 are shown positioned adjacent to the distal end of the braided layer 704, in the distal-most cells 731 of the braid. In other fabrication methods, radiopaque markers 729 can be positioned in cells anywhere along the length of braided layer 704.

The radiopaque features shown in FIG. 56 and FIG. 57 have defined features (such as the chevrons 723 of FIG. 56 or the diamond-like shape of the features 729). Some fabrication methods can include cutting one or more radiopaque features from a sheet of radiopaque metal. The radiopaque features can be cut using a laser, a sharp tool, or chemical etching, for example.

In some aspects, a radiopaque feature that does not directly contact the braided layer may still be indirectly coupled to a filament of the braided layer. For example, the fabrication methods can include forming an aperture in the radiopaque feature, stringing a cord or suture through the aperture, and coupling the cord or suture to the braided layer. Or, for example, the radiopaque feature can be welded, crimped, or bonded to extensions that are then welded, crimped, or bonded to the braided layer.

Some methods include positioning a filament 733 of a braided layer 704 within a lumen of a radiopaque feature 735, as shown in FIG. 58. This method could include coiling a radiopaque metal around filament 733 to form radiopaque coil 735. In FIG. 58, the radiopaque coil 735 is shown positioned adjacent to the distal end 719 of the braided layer 704. In other fabrication methods, one or more radiopaque features such as the radiopaque coil 735 can be positioned anywhere along the length of braided layer 704. Alternatively, or in addition, the fabrication methods can include sliding a radiopaque tube 736 over a filament 733 of a braided layer 704, as shown in FIG. 61. A radiopaque coil 735 or a radiopaque tube 736 can be crimped, swaged, pressed, or welded to braided layer 704.

Some methods include directly coupling the radiopaque feature the braided layer. For example, one example method can include a step of applying a radiopaque coating to one or more filaments of a braided layer. Some methods could include forming a groove within a radiopaque feature, and press fitting the groove of the radiopaque feature onto a filament 733 of a braided layer 704. One or more press-fit radiopaque features can be positioned anywhere along the length of braided layer 704, and in conjunction with any other radiopaque feature.

FIG. 59 shows radiopaque beads 737 directly coupled to a braided layer 741. As part of the fabrication process of this sheath, a braided tube is cut perpendicular to its longitudinal axis to form a free cut braided layer 741 prior to applying the radiopaque beads 737. Free distal ends of two circumferentially adjacent filaments, such as 733a and 733b, are joined by a radiopaque bead 737. The radiopaque beads may be welded or crimped onto the distal ends of filaments 733a, 733b.

A method of delivering a medical device can include the following steps. An expandable sheath is inserted, at least partially, into the vasculature of a patient. The expandable sheath can include, at least, a first polymeric layer, a braided layer radially outward of the first polymeric layer, a radiopaque feature positioned radially outward of the first polymeric layer, and a second polymeric layer positioned radially outward of the braided layer and the radiopaque feature. The second polymeric layer can be coupled to the first polymeric layer such that the braided layer and the radiopaque feature are encapsulated between the first and second polymeric layers. The method can further include visualizing the position of a radiopaque feature of the sheath within the vasculature using an imaging modality and advancing a medical device through an inner lumen defined by the sheath. The medical device applies an outward radial force on an inner surface of the sheath. The method can further include locally expanding the sheath from a collapsed state to a locally expanded state at the longitudinal position of the advancing medical device. The locally expanded portion of the sheath collapses at least partially back to the collapsed state after passage of the medical device.

In one example method of delivering a medical device, an expandable sheath 701 is inserted, at least partially, into the vasculature of the patient. By some methods, the sheath 701 is inserted at the femoral artery. Imaging modalities can be used to visualize the sheath 701 inside the vasculature via a radiopaque foil layer 705, such as the one shown in FIG. 48 and FIG. 49. For example, fluoroscopic imaging uses X-ray to obtain real-time moving images of radiopaque (X-ray blocking) objects. A medical device can be advanced through an inner lumen of the visualized sheath 701. The medical device applies an outward radial force on an inner surface of the sheath 701 and the sheath 701 expands locally at the position of the medical device. During expansion, a plurality of longitudinally extending folds in the layers of the sheath, including the foil layer 705, at least partially unfold. The sheath wall thickness decreases as a result of this unfolding, which results in a decreased radiopacity of the sheath at the locally expanded location. If desired, this decrease in radiopacity can be tracked as the device advances through the sheath and, in some methods, the decreased radiopacity can be quantified using imaging modalities.

The expanded portion of the sheath 701 collapses back, at least partially, to its original collapsed state after the medical device has passed. The local collapse of the sheath includes a partial refolding of the plurality of longitudinally extending folds. In some methods, the local collapse can be facilitated by direction of an inward radial force on the foil layer 705 and/or other layers of the sheath 701. For example, a tubular outer elastic layer can compress the inner layers of sheath 701, including foil layer 705, back into an at least partially folded configuration. Or, a self-collapsing braided layer can impose an inwardly directed radial force to facilitate the local collapse of the sheath 701. The foil layer 705, being coupled to the self-collapsing braided layer, folds back into an at least partially folded configuration.

In some methods of delivering a medical device, locally expanding the sheath from a collapsed state to a locally expanded state comprises changing the pitch of a helically wound radiopaque cord, such as cord 717 shown in FIG. 55. In some methods, locally expanding the sheath from a collapsed state to a locally expanded state comprises increasing the inner angle α of each chevron 723 in a circumferentially extending series of chevrons, such as those shown in FIG. 56. In some methods, locally expanding the sheath from a collapsed state to a locally expanded state comprises shifting filaments of the braided layer, wherein the radiopaque feature is coupled to and moves with at least one filament of the braided layer. For example, the radiopaque coil 735 shown in FIG. 58 or radiopaque tube 736 of FIG. 62 would shift with the filament 733 to which it is attached during expansion of the sheath. Some methods of delivering a medical device, further include preventing distal ends of two circumferentially adjacent filaments of the braided layer from separating using the radiopaque feature, such as the radiopaque bead(s) 737 shown in FIG. 59.

### General Considerations

For purposes of this description, certain aspects, advantages, and novel features of the aspects of this disclosure are described herein. The disclosed methods, apparatus, and systems should not be construed as being limiting in any way. Instead, the present disclosure is directed toward all novel and nonobvious features and aspects of the various disclosed aspects, alone and in various combinations and sub-combinations with one another. The methods, apparatus, and systems are not limited to any specific aspect or feature or combination thereof, nor do the disclosed aspects require that any one or more specific advantages be present or problems be solved.

Features, integers, characteristics, compounds, chemical moieties, or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive. The disclosure is not restricted to the details of any foregoing aspects. The disclosure extends to any novel one, or any novel combination, of the features disclosed in this specification (including any accompanying claims, abstract, and drawings), or to any novel one, or any novel combination, of the steps of any method or process so disclosed.

Although the operations of some of the disclosed aspects are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the terms "coupled" and "associated" generally mean electrically, electromagnetically, and/or physically (e.g., mechanically or chemically) coupled or linked and does not exclude the presence of intermediate elements between the coupled or associated items absent specific contrary language.

Unless otherwise indicated, all numbers expressing dimensions, quantities of components, molecular weights, percentages, temperatures, forces, times, and so forth, as used in the specification or claims, are to be understood as being modified by the term "about." Accordingly, unless otherwise indicated, implicitly or explicitly, the numerical parameters set forth are approximations that can depend on the desired properties sought and/or limits of detection under test conditions/methods familiar to those of ordinary skill in the art. When directly and explicitly distinguishing aspects from discussed prior art, the aspect numbers are not approximates unless the word "about" is recited. Furthermore, not all alternatives recited herein are equivalents.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. The terms "about" and "approximately" are defined as being "close to" as understood by one of ordinary skill in the art. In one non-limiting aspect the terms are defined to be within 10%. In another non-limiting aspect, the terms are defined to be within 5%. In still another non-limiting aspect, the terms are defined to be within 1%.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device toward the user, while distal motion of the device is motion of the device away from the user. The terms "longitudinal" and "axial" refer to an axis extending in the proximal and distal directions, unless otherwise expressly defined.

In view of the many possible aspects to which the principles of the disclosed technology may be applied, it should be recognized that the illustrated aspects are only preferred examples and should not be taken as limiting the scope of the disclosure. Rather, the scope of the disclosure is at least as broad as the following claims. We therefore claim all that comes within the scope of these claims.

## Claims

1. An expandable sheath (701) for deploying a medical device, comprising:
a sheath wall (703) comprising a tubular first polymeric layer (707) defining an inner lumen and a foil layer (705) with radiopaque properties positioned radially outward of and extending circumferentially around the first polymeric layer (707), the sheath wall (703) further comprising a plurality of longitudinally extending folds (763) when the sheath is in a collapsed state;
wherein when a medical device passing through the inner lumen of the sheath applies an outward radial force on the sheath wall (703), the sheath wall (703) expands radially from the collapsed state to an expanded state by at least partially unfolding the plurality of longitudinally extending folds (763); and
wherein the at least partial unfolding of the plurality of longitudinally extending folds (763) causes a decrease in sheath wall (703) thickness.

2. The expandable sheath (701) of claim 1, wherein the decrease in the wall thickness (T) of the sheath can be detected by an imaging modality as a decrease in radiopacity.

3. The expandable sheath (701) of either claim 1 or claim 2, wherein the thickness of the foil layer (705) is from about 10 microns to about 30 microns.

4. The expandable sheath (701) of any one of claims 1-3, further comprising a second polymeric layer (709) positioned radially outward of the foil layer (705).

5. The expandable sheath (701) of any one of claims 1-4, wherein the sheath wall (703) further comprises a braided layer (704) positioned radially between the first polymeric layer (707) and the foil layer (705), the braided layer (704) comprising a plurality of filaments braided together.

6. The expandable sheath (701) of any one of claims 1-3 and 5, wherein the foil layer (705) is the radially outermost layer of the sheath wall (703).

7. The expandable sheath (701) of claim 6, wherein the sheath wall (703) further comprises a second polymeric layer (709) positioned radially inward of the foil layer (705) and a braided layer (704) positioned radially inward of the second polymeric layer (709).

8. The expandable sheath (701) of any one of claims 1-7, wherein the foil layer (705) comprises openings (773);
preferably wherein the first polymeric layer (707) is coupled to a second polymeric layer (709) through the openings (773) of the foil layer (705);
more preferably wherein the first polymeric layer (707) is further coupled to the second polymeric layer (709) between filaments of a braided layer (704).

9. The expandable sheath (701) of any one of claims 1-8, wherein the foil layer (705) comprises a radiopaque metal.

10. The expandable sheath (701) of any one of claims 1-9, wherein the foil layer (705) comprises a polymer material.

11. The expandable sheath (701) of any one of claims 1-10, wherein the foil layer (705) comprises a polymer material mixed with a radiopaque powder.

12. The expandable sheath (701) of any one of claims 1-11, wherein the foil layer (705) comprises a polymer sheet surface-treated with a radiopaque powder.

13. The expandable sheath (701) of any one of claims 1-12, wherein the longitudinally extending folds (763) create a plurality of circumferentially spaced ridges and a plurality of circumferentially spaced valleys, and wherein, as the medical device is passed through the sheath, the ridges and valleys level out to allow the sheath wall (703) to radially expand.

14. The expandable sheath (701) of any one of claims 1-13, wherein the expandable sheath (701) further comprises an outer cover (400) formed of a heat shrink material and extending over a longitudinal portion of the sheath wall (703), the outer cover (400) comprising one or more longitudinally extending slits, weakened portions, or scorelines (406).

15. The expandable sheath (701) of any one of claims 1-14, wherein the sheath further comprises an elastic outer layer that applies an inward radial force on the sheath wall (703), biasing the sheath toward the collapsed state.

## Patentansprüche

1. Expandierbare Hülle (701) zum Einsetzen einer medizinischen Vorrichtung, umfassend:
eine Hüllenwand (703), umfassend eine röhrenförmige erste Polymerschicht (707), die ein inneres Lumen definiert, und eine Folienschicht (705) mit röntgendichten Eigenschaften, die radial außerhalb der ersten Polymerschicht (707) positioniert ist und sich um deren Umfang herum erstreckt, wobei die Hüllenwand (703) ferner eine Vielzahl sich longitudinal erstreckender Faltungen umfasst, wenn sich die Hülle in einem zusammengeklappten Zustand befindet;
wobei sich die Hüllenwand (703), wenn ein medizinisches Gerät, das durch das innere Lumen der Hülle hindurchgeht, eine nach außen gerichtete Radialkraft auf die Hüllenwand (703) ausübt, radial von dem zusammengeklappten Zustand zu einem expandierten Zustand ausdehnt, indem sich die Vielzahl der sich longitudinal erstreckenden Faltungen (763) zumindest teilweise entfaltet; und
wobei das zumindest teilweise Entfalten der Vielzahl der sich longitudinal erstreckenden Faltungen (763) eine Verringerung der Stärke der Hüllenwand (703) bewirkt.

2. Expandierbare Hülle (701) gemäß Anspruch 1, wobei die Verringerung der Wandstärke (T) der Hülle durch ein bildgebendes Verfahren als Verringerung der Röntgenopazität detektiert werden kann.

3. Expandierbare Hülle (701) gemäß Anspruch 1 oder Anspruch 2, wobei die Stärke der Folienschicht (705) zwischen etwa 10 Mikrometern und etwa 30 Mikrometern liegt.

4. Expandierbare Hülle (701) gemäß einem der Ansprüche 1 bis 3, ferner umfassend eine zweite Polymerschicht (709), die radial außerhalb der Folienschicht (705) angeordnet ist.

5. Expandierbare Hülle (701) gemäß einem der Ansprüche 1 bis 4, wobei die Hüllenwand (703) ferner eine geflochtene Schicht (704) umfasst, die radial zwischen der ersten Polymerschicht (707) und der Folienschicht (705) angeordnet ist, wobei die geflochtene Schicht (704) eine Vielzahl von miteinander verflochtenen Filamenten umfasst.

6. Expandierbare Hülle (701) gemäß einem der Ansprüche 1 bis 3 und 5, wobei die Folienschicht (705) die radial äußerste Schicht der Hüllenwand (703) ist.

7. Expandierbare Hülle (701) gemäß Anspruch 6, wobei die Hüllenwand (703) ferner eine zweite Polymerschicht (709), die radial innerhalb der Folienschicht (705) angeordnet ist, und eine geflochtene Schicht (704), die radial innerhalb der zweiten Polymerschicht (709) angeordnet ist, umfasst.

8. Expandierbare Hülle (701) gemäß einem der Ansprüche 1 bis 7, wobei die Folienschicht (705) Öffnungen (773) umfasst;
wobei, vorzugsweise, die erste Polymerschicht (707) an eine zweite Polymerschicht (709) durch die Öffnungen (773) der Folienschicht (705) gekoppelt ist;
wobei, noch bevorzugter, die erste Polymerschicht (707) ferner an die zweite Polymerschicht (709) zwischen Filamenten einer geflochtenen Schicht (704) gekoppelt ist.

9. Expandierbare Hülle (701) gemäß einem der Ansprüche 1 bis 8, wobei die Folienschicht (705) ein röntgendichtes Metall umfasst.

10. Expandierbare Hülle (701) gemäß einem der Ansprüche 1 bis 9, wobei die Folienschicht (705) ein Polymermaterial umfasst.

11. Expandierbare Hülle (701) gemäß einem der Ansprüche 1 bis 10, wobei die Folienschicht (705) ein Polymermaterial gemischt mit einem röntgendichten Pulver umfasst.

12. Expandierbare Hülle (701) gemäß einem der Ansprüche 1 bis 11, wobei die Folienschicht (705) eine Polymerfolie, deren Oberfläche mit einem röntgendichten Pulver behandelt ist, umfasst.

13. Expandierbare Hülle (701) gemäß einem der Ansprüche 1 bis 12, wobei die sich longitudinal erstreckenden Faltungen (763) eine Vielzahl von in Umfangsrichtung beabstandeten Erhebungen und eine Vielzahl von in Umfangsrichtung beabstandeten Vertiefungen bilden, und wobei sich die Erhebungen und Vertiefungen beim Durchführen der medizinischen Vorrichtung durch die Hülle ausgleichen, damit sich die Hüllenwand (703) radial ausdehnen kann.

14. Expandierbare Hülle (701) gemäß einem der Ansprüche 1 bis 13, wobei die expandierbare Hülle (701) ferner eine Außenhülle (400) umfasst, die aus einem wärmeschrumpfbaren Material gebildet ist und sich über einen Längsabschnitt der Hüllenwand (703) erstreckt, wobei die Außenhülle (400) einen oder mehrere sich longitudinal erstreckende Schlitze, geschwächte Abschnitte oder Kerblinien (406) umfasst.

15. Expandierbare Hülle (701) gemäß einem der Ansprüche 1 bis 14, wobei die Hülle ferner eine elastische Außenschicht umfasst, die eine nach innen gerichtete Radialkraft auf die Hüllenwand (703) ausübt, wodurch sie die Hülle in Richtung des zusammengeklappten Zustands verschiebt.

## Revendications

1. Gaine extensible (701) destinée à déployer un dispositif médical, comprenant :
une paroi (703) de gaine comprenant une première couche polymère tubulaire (707) définissant une lumière interne et une couche de feuille (705) présentant des propriétés radio-opaques positionnée radialement vers l'extérieur par rapport à la première couche polymère (707) et s'étendant de manière circonférentielle autour de celle-ci, la paroi (703) de gaine comprenant en outre une pluralité de plis (763) s'étendant longitudinalement lorsque la gaine est dans un état replié ;
dans laquelle, lorsqu'un dispositif médical passant à travers la lumière interne de la gaine applique une force radiale vers l'extérieur sur la paroi (703) de gaine, la paroi (703) de gaine s'étend radialement à partir de l'état replié vers un état étendu en dépliant au moins partiellement la pluralité de plis (763) s'étendant longitudinalement ; et
le dépliage au moins partiel de la pluralité de plis (763) s'étendant longitudinalement provoquant une diminution de l'épaisseur de la paroi (703) de gaine.

2. Gaine extensible (701) selon la revendication 1, dans laquelle la diminution de l'épaisseur de paroi (T) de la gaine peut être détectée par une modalité d'imagerie en tant que diminution de la radio-opacité.

3. Gaine extensible (701) selon soit la revendication 1, soit la revendication 2, dans laquelle l'épaisseur de la couche de feuille (705) est d'environ 10 microns à environ 30 microns.

4. Gaine extensible (701) selon l'une quelconque des revendications 1 à 3, comprenant en outre une seconde couche polymère (709) positionnée radialement vers l'extérieur par rapport à la couche de feuille (705).

5. Gaine extensible (701) selon l'une quelconque des revendications 1 à 4, dans laquelle la paroi (703) de gaine comprend en outre une couche tressée (704) positionnée radialement entre la première couche polymère (707) et la couche de feuille (705), la couche tressée (704) comprenant une pluralité de filaments tressés ensemble.

6. Gaine extensible (701) selon l'une quelconque des revendications 1 à 3 et 5, dans laquelle la couche de feuille (705) est la couche radialement la plus à l'extérieur de la paroi (703) de gaine.

7. Gaine extensible (701) selon la revendication 6, dans laquelle la paroi (703) de gaine comprend en outre une seconde couche polymère (709) positionnée radialement vers l'intérieur par rapport à la couche de feuille (705) et une couche tressée (704) positionnée radialement vers l'intérieur par rapport à la seconde couche polymère (709).

8. Gaine extensible (701) selon l'une quelconque des revendications 1 à 7, dans laquelle la couche de feuille (705) comprend des ouvertures (773) ;
de préférence dans laquelle la première couche polymère (707) est accouplée à une seconde couche polymère (709) à travers les ouvertures (773) de la couche de feuille (705) ;
plus préférablement dans laquelle la première couche polymère (707) est en outre accouplée à la seconde couche polymère (709) entre des filaments d'une couche tressée (704).

9. Gaine extensible (701) selon l'une quelconque des revendications 1 à 8, dans laquelle la couche de feuille (705) comprend un métal radio-opaque.

10. Gaine extensible (701) selon l'une quelconque des revendications 1 à 9, dans laquelle la couche de feuille (705) comprend un matériau polymère.

11. Gaine extensible (701) selon l'une quelconque des revendications 1 à 10, dans laquelle la couche de feuille (705) comprend un matériau polymère mélangé avec une poudre radio-opaque.

12. Gaine extensible (701) selon l'une quelconque des revendications 1 à 11, dans laquelle la couche de feuille (705) comprend une feuille de polymère traitée en surface par une poudre radio-opaque.

13. Gaine extensible (701) selon l'une quelconque des revendications 1 à 12, dans laquelle les plis (763) s'étendant longitudinalement créent une pluralité de crêtes espacées de manière circonférentielle et une pluralité de creux espacés de manière circonférentielle et dans laquelle, pendant le passage du dispositif médical à travers la gaine, les crêtes et les creux s'aplanissent afin de permettre à la paroi (703) de gaine de s'étendre radialement.

14. Gaine extensible (701) selon l'une quelconque des revendications 1 à 13, dans laquelle la gaine extensible (701) comprend en outre un revêtement externe (400) formé d'un matériau thermorétractable et s'étendant sur une partie longitudinale de la paroi (703) de gaine, le revêtement externe (400) comprenant une ou plusieurs fentes, parties affaiblies ou lignes de rupture (406) s'étendant longitudinalement.

15. Gaine extensible (701) selon l'une quelconque des revendications 1 à 14, dans laquelle la gaine comprend en outre une couche externe élastique qui applique une force radiale vers l'intérieur sur la paroi (703) de gaine, sollicitant la gaine vers l'état replié.
